(19) Europäisches Patentamt
European Patent Office
Office européen des brevets

(11) EP 3 042 656 A1

(12) EUROPEAN PATENT APPLICATION
published in accordance with Art. 153(4) EPC

(43) Date of publication:
**13.07.2016 Bulletin 2016/28**

(21) Application number: **14839861.3**

(22) Date of filing: **02.09.2014**

(51) Int Cl.:
***A61K 31/44*** *(2006.01)* ***A61K 31/4436*** *(2006.01)*
***A61K 31/444*** *(2006.01)* ***A61P 11/00*** *(2006.01)*

(86) International application number:
**PCT/JP2014/072987**

(87) International publication number:
**WO 2015/030250 (05.03.2015 Gazette 2015/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.09.2013 JP 2013181526**

(71) Applicant: **UBE Industries, Ltd.**
**Ube-shi**
**Yamaguchi 755-8633 (JP)**

(72) Inventors:
- **YONEDA, Kenji**
  **Ube-shi**
  **Yamaguchi 755-8633 (JP)**
- **SHIBAKAWA, Nobuhiko**
  **Ube-shi**
  **Yamaguchi 755-8633 (JP)**
- **KATSUBE, Tetsushi**
  **Ube-shi**
  **Yamaguchi 755-8633 (JP)**
- **KANDA, Tomoko**
  **Ube-shi**
  **Yamaguchi 755-8633 (JP)**
- **ITO, Koji**
  **Ube-shi**
  **Yamaguchi 755-8633 (JP)**
- **YAMAMOTO, Kiyoshi**
  **Ube-shi**
  **Yamaguchi 755-8633 (JP)**
- **SHINOHARA, Masaru**
  **Ube-shi**
  **Yamaguchi 755-8633 (JP)**
- **IWASE, Noriaki**
  **Ube-shi**
  **Yamaguchi 755-8633 (JP)**
- **USHIYAMA, Shigeru**
  **Ube-shi**
  **Yamaguchi 755-8633 (JP)**

(74) Representative: **Hoffmann Eitle**
**Patent- und Rechtsanwälte PartmbB**
**Arabellastraße 30**
**81925 München (DE)**

# (54) PHARMACEUTICAL COMPOSITION FOR TREATMENT AND/OR PREVENTION OF PULMONARY DISEASE

(57) Provided is a pharmaceutical composition containing, as an active ingredient thereof, a substituted biaryl compound represented by general formula (I), wherein, $R^1$, W, $R^2$ and Z are as defined in the claims and description, or a pharmacologically acceptable salt thereof. The pharmaceutical composition of the present invention is useful as a therapeutic drug and/or prophylactic drug for chronic obstructive pulmonary disease due to having an excellent anti-inflammatory effect.

EP 3 042 656 A1

## Description

### TECHNICAL FIELD

The present invention relates to a pharmaceutical composition for the treatment and/or prevention of pulmonary disease containing, as an active ingredient thereof, a novel substituted biaryl compound or a pharmacologically acceptable salt thereof.

### BACKGROUND ART

Chronic obstructive pulmonary disease is a disorder composed of a diverse range of pathological conditions including structural and functional changes in the lungs, and is characterized by emphysema, chronic bronchitis caused by increased secretion of mucus in the bronchi and irreversible, persistent airway obstruction. This disease is caused by smoking, gases associated with air pollution or the presence of harmful fine particles, and leads to inflammation of the bronchi and destruction of alveoli. As a result, patients present with symptoms such as coughing, expulsion of phlegm or shortness of breath, and in advanced cases, may progress to respiratory failure due to hypoxemia.

Drugs used for the treatment of chronic obstructive pulmonary disease consist primarily of anticholinergic drugs, β2 agonists, theophylline preparations and other bronchodilators, and although other drugs such as expectorants or steroids are also used, a safe and established therapeutic drug has yet to be found. Thus, there is a desire for a more effective drug for the treatment of chronic obstructive pulmonary disease that is associated with minimal adverse side effects.

On the other hand, prostaglandin $E_2$ (hereinafter, abbreviated as "$PGE_2$") has a wide variety of physiological activities as a metabolic product in the arachidonic acid cascade, and acts as an agonist against the four receptors of EP1, EP2, EP3 and EP4. $PGE_2$ is involved in numerous inflammatory reactions, and in addition to having a prophlogistic effect such as upregulation of vascular permeability, release of various types of inflammation mediators, induction of inflammatory cells/immune cells and promotion of vascularization, $PGE_2$ has also been reported to demonstrate an anti-inflammatory effect mediated by EP2 and/or EP4 receptors (see Non-Patent Document 1).

It has previously been disclosed that a prostanoid-based compound exhibiting an EP2 antagonistic effect is useful for the prevention and/or treatment of respiratory diseases including chronic obstructive pulmonary disease (see Patent Document 1). In addition, non-prostanoid-based compounds exhibiting an EP2 antagonistic effect are also known (see Patent Documents 2 to 7), and among these, various diseases exemplified as medicinal applications of the compounds described in Patent Documents 2 to 7 include chronic obstructive pulmonary disease.

### PRIOR ART DOCUMENTS

#### Patent Documents

Patent Document 1: WO2006/043655
Patent Document 2: WO2009/113600
Patent Document 3: WO2011/030868
Patent Document 4: WO2011/030871
Patent Document 5: WO2011/030872
Patent Document 6: WO2011/030873
Patent Document 7: WO2011/078303

#### Non-Patent Documents

Non-Patent Document 1: British Journal of Pathology, 122, 149 (1997)

### SUMMARY OF THE INVENTION

#### Problems to be Solved by the Invention

However, examples of specific pharmacological studies indicating that the compounds described in the aforementioned Patent Document 2 to 7 have anti-inflammatory effects and are useful for chronic obstructive pulmonary disease are not described in any of the prior art documents. Moreover, none of the aforementioned prior art documents

describe examples of the sulfonamide compound according to the present invention having, as a partial structure thereof, a biaryl group in which a specific substituent is substituted at a specific site.

The inventors of the present invention conducted intensive research for the purpose of providing a pharmaceutical composition that is useful for the treatment and/or prevention of chronic obstructive pulmonary disease. As a result, since a compound in which a specific substituent of a specific length is introduced at a specific site of a terminal aryl group of a sulfonamide compound having a biaryl group has excellent anti-inflammatory action, such as action that inhibits the production of inflammatory cytokines, it was found to be useful as a therapeutic and/or prophylactic drug (and preferably as a therapeutic drug) for chronic obstructive pulmonary disease in particular, thereby leading to completion of the present invention.

The present invention provides a pharmaceutical composition containing, as an active ingredient thereof, a substituted biaryl compound, or a pharmacologically acceptable salt thereof, that has an EP2 antagonistic effect and an excellent anti-inflammatory effect, and is useful as a therapeutic drug and/or prophylactic drug (and preferably as a therapeutic drug) for chronic obstructive pulmonary disease in particular.

Means for Solving the Problems

The present invention provides the following.

(1) A pharmaceutical composition for treatment and/or prevention of chronic obstructive pulmonary disease, comprising a substituted biaryl compound represented by general formula (I):

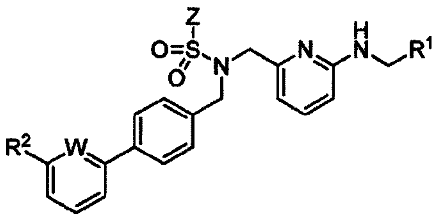

(I)

wherein,

$R^1$ represents a protected or unprotected carboxy group,
W represents a nitrogen atom or -CH= group,
$R^2$ represents an ethoxy group, 1-propenyl group or 1-propynyl group, and
Z represents a phenyl group, 3-fluorophenyl group, pyridin-2-yl group, pyridin-3-yl group, thiophen-2-yl group or thiophen-3-yl group

or a pharmacologically acceptable salt thereof.

(2) The pharmaceutical composition described in (1), wherein, in general formula (I), $R^1$ represents a carboxy group or $C_1$-$C_6$ alkoxycarbonyl group.

(3) The pharmaceutical composition described in (1), wherein, in general formula (I), $R^1$ represents a carboxy group, ethoxycarbonyl group, isopropoxycarbonyl group or hexyloxycarbonyl group.

(4) The pharmaceutical composition described in (1), wherein, in general formula (I),
$R^1$ represents a carboxy group, ethoxycarbonyl group, isopropoxycarbonyl group or hexyloxycarbonyl group,
W represents a nitrogen atom or -CH= group,
$R^2$ represents a 1-propenyl group or 1-propynyl group, and
Z represents a phenyl group, 3-fluorophenyl group, pyridin-2-yl group, pyridin-3-yl group, thiophen-2-yl group or thiophen-3-yl group.

(5) The pharmaceutical composition described in (1), wherein the substituted biaryl compound represented by general formula (I) is:

ethyl (6-{[3'-(1-propenyl)biphenyl-4-ylmethyl](pyridin-2-ylsulfonyl)aminomethyl}pyridin-2-ylamino)acetate,
(6-{[3'-(1-propenyl)biphenyl-4-ylmethyl](pyridin-2-ylsulfonyl)aminomethyl}-pyridin-2-ylamino)acetic acid,
ethyl (6-{[3'-(1-propynyl)biphenyl-4-ylmethyl](pyridin-2-ylsulfonyl)aminomethyl}pyridin-2-ylamino)acetate,
(6-{[3'-(1-propynyl)biphenyl-4-ylmethyl](pyridin-2-ylsulfonyl)aminomethyl}-pyridin-2-ylamino)acetic acid,
ethyl (6-{[3'-(1-propynyl)biphenyl-4-ylmethyl](pyridin-3-ylsulfonyl)aminomethyl}pyridin-2-ylamino)acetate,
(6-{[3'-(1-propynyl)biphenyl-4-ylmethyl](pyridin-3-ylsulfonyl)aminomethyl}-pyridin-2-ylamino)acetic acid,
{6-[(3'-ethoxybiphenyl-4-ylmethyl)(pyridin-2-ylsulfonyl)aminomethyl]-pyridin-2-ylamino}acetic acid,

hexyl {6-[(3'-ethoxybiphenyl-4-ylmethyl)(pyridin-2-ylsulfonyl)aminomethyl]-pyridin-2-ylamino}acetate,
{6-[(3'-ethoxybiphenyl-4-ylmethyl)(pyridin-3-ylsulfonyl)aminomethyl]-pyridin-2-ylamino}acetic acid,
{6-[(benzenesulfonyl)(3'-ethoxybiphenyl-4-ylmethyl)aminomethyl]-pyridin-2-ylamino}acetic acid,
{6-[(3'-ethoxybiphenyl-4-ylmethyl)(thiophen-2-ylsulfonyl)aminomethyl]-pyridin-2-ylamino}acetic acid,
(6-{[4-(6-ethoxypyridin-2-yl)benzyl](pyridin-2-ylsulfonyl)aminomethyl}-pyridin-2-ylamino)acetic acid,
ethyl (6-{[3'-(1-propynyl)biphenyl-4-ylmethyl](thiophen-2-ylsulfonyl)-aminomethyl}pyridin-2-ylamino)acetate,
(6-{[3'-(1-propynyl)biphenyl-4-ylmethyl](thiophen-2-ylsulfonyl)-aminomethyl}pyridin-2-ylamino)acetic acid,
ethyl (6-{(benzenesulfonyl)[3'-(1-propynyl)biphenyl-4-ylmethyl]-aminomethyl}pyridin-2-ylamino)acetate,
(6-{(benzenesulfonyl)[3'-(1-propynyl)biphenyl-4-ylmethyl]aminomethyl}-pyridin-2-ylamino)acetic acid,
ethyl (6-{[3'-(1-propynyl)biphenyl-4-ylmethyl](thiophen-3-ylsulfonyl)-aminomethyl}pyridin-2-ylamino)acetate,
(6-{[3'-(1-propynyl)biphenyl-4-ylmethyl](thiophen-3-ylsulfonyl)-aminomethyl}pyridin-2-ylamino)acetic acid,
(6-{(3-fluorobenzenesulfonyl)[3'-(1-propynyl)biphenyl-4-ylmethyl]-aminomethyl}pyridin-2-ylamino)acetic acid,
or
isopropyl (6-{[3'-(1-propynyl)biphenyl-4-ylmethyl](pyridin-2-ylsulfonyl)aminomethyl}pyridin-2-ylamino)acetate.

Effect of the Invention

The substituted biaryl compound represented by general formula (I), or the pharmacologically acceptable salt thereof, of the present invention is effective for chronic obstructive pulmonary disease due to having an EP2 antagonistic effect and an excellent anti-inflammatory effect. Thus, a pharmaceutical composition containing the substituted biaryl compound represented by general formula (I), or the pharmacologically acceptable salt thereof, of the present invention as an active ingredient thereof is useful as pharmaceuticals, and especially as a therapeutic and/or prophylactic drug (and preferably as a therapeutic drug) for chronic obstructive pulmonary disease.

MODE FOR CARRYING OUT THE INVENTION

Preferred embodiments of each substituent in the substituted biaryl compound represented by the aforementioned general formula (I) are indicated below.

The protected or unprotected carboxy group represented by $R^1$ of general formula (I) refers to a carboxy group or a carboxy group protected by a protective group. Examples of such a protective group include ester-type protective groups. Examples of the partial structure of the ester-type protective group include $C_1$-$C_{12}$ alkyl groups, such as a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group or dodecyl group; $C_7$-$C_{18}$ aralkyl groups, such as a benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group or phenyldodecyl group; $C_1$-$C_4$ alkyl groups substituted with a $C_2$-$C_5$ alkanoyloxy group, such as an acetoxymethyl group, 1-acetoxyethyl group, 1-acetoxypropyl group, 1-acetoxybutyl group, propanoyloxymethyl group, 1-propanoyloxyethyl group, butanoyloxymethyl group, 1-butanoyloxyethyl group, pivaloyloxymethyl group, 1-pivaloyloxyethyl group, 1-pivaloyloxypropyl group or 1-pivaloyloxybutyl group; $C_1$-$C_4$ alkyl groups substituted with a ($C_1$-$C_4$ allcoxy)carbonyloxy group, such as a methoxycarbonyloxymethyl group, 1-methoxycarbonyloxyethyl group, ethoxycarbonyloxymethyl group, 1-ethoxycarbonyloxyethyl group, propoxycarbonyloxymethyl group, 1-propoxycarbonyloxyethyl group, isopropoxycarbonyloxymethyl group, 1-isopropoxycarbonyloxyethyl group, butoxycarbonyloxymethyl group, 1-butoxycarbonyloxyethyl group, tert-butoxycarbonyloxymethyl group or 1-tert-butoxycarbonyloxyethyl group; N,N-dialkylaminocarbonylalkyl groups, such as an N,N-dimethylaminocarbonylmethyl group or N,N-diethylaminocarbonylmethyl group; 2-(N,N-dialkylamino)ethyl groups, such as a 2-(N,N-dimethylamino)ethyl group or 2-(N,N-diethylamino)ethyl group; $C_1$-$C_4$ alkyl groups substituted with a 5-membered or 6-membered saturated heteromonocyclic group containing 1 or 2 hetero atoms selected from N, O and S, such as a 2-(morpholin-4-yl)ethyl group, 2-piperidinoethyl group or 2-(4-methylpiperidino)ethyl group; and groups that are readily deprotected in vivo and can be converted to a carboxy group, such as a (5-methyl-2-oxo-1,3-dioxolen-4-yl)methyl group or (5-phenyl-2-oxo-1,3-dioxolen-4-yl)methyl group. The partial structure of the ester-type protective group is preferably a $C_1$-$C_{12}$ alkyl group, $C_7$-$C_{18}$ aralkyl group, $C_1$-$C_2$ alkyl group substituted with a $C_2$-$C_5$ alkanoyloxy group, $C_1$-$C_2$ alkyl group substituted with a ($C_1$-$C_4$ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group. The partial structure of the ester-type protective group is more preferably a $C_1$-$C_6$ alkyl group, and particularly preferably an ethyl group, isopropyl group or hexyl group.

Therefore, in general formula (I) of the present invention, $R^1$ is preferably a carboxy group or a $C_1$-$C_6$ alkoxycarbonyl group. In a specific embodiment of general formula (I) of the present invention, $R^1$ is a carboxy group, ethoxycarbonyl group, isopropoxycarbonyl group or hexyloxycarbonyl group.

In general formula (I) of the present invention, W is a nitrogen atom or -CH= group. That is, in general formula (I) of the present invention, the aromatic ring containing W is a pyridine ring or benzene ring. In a specific embodiment of general formula (I) of the present invention, W is a -CH= group. In another specific embodiment of general formula (I) of the present invention, W is a nitrogen atom.

In general formula (I) of the present invention, $R^2$ is an ethoxy group, 1-propenyl group or 1-propynyl group. In a specific embodiment of general formula (I) of the present invention, $R^2$ is an ethoxy group. In another specific embodiment of general formula (I) of the present invention, $R^2$ is a 1-propenyl group or 1-propynyl group.

In general formula (I) of the present invention, Z is a phenyl group, 3-fluorophenyl group, pyridin-2-yl group, pyridin-3-yl group, thiophen-2-yl group or thiophen-3-yl group. In a specific embodiment of general formula (I) of the present invention, Z is a phenyl group, 3-fluorophenyl group, pyridin-2-yl group or pyridin-3-yl group, and preferably a phenyl group, pyridin-2-yl group or pyridin-3-yl group. In another specific embodiment of general formula (I) of the present invention, Z is a thiophen-2-yl group or thiophen-3-yl group, and preferably a thiophen-2-yl group.

When the compound of general formula (I) of the present invention has a geometrical isomer or a rotational isomer, these isomers are also included in the scope of the present invention, and when the compound has a proton tautomer, such tautomer is also included in the scope of the present invention.

Although the compound represented by general formula (I) of the present invention can be converted to a pharmacologically acceptable salt by a conventional method as necessary, the pharmacologically acceptable salt can be also directly separated from the reaction mixture as a salt.

The compound represented by general formula (I) of the present invention is converted to a pharmacologically acceptable acid addition salt by treating it with an acid. Examples of such a salt include, for example, inorganic acid salts, such as a hydrochloride, hydrobromide, hydroiodide, nitrate, sulfate or phosphate; and organic acid salts, such as an acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate or aspartate.

When $R^1$ in the compound represented by general formula (I) of the present invention is a carboxy group, the compound is converted to a pharmacologically acceptable basic salt by treating it with a base. Examples of such a salt include metal salts, such as a sodium salt, potassium salt, calcium salt or magnesium salt; inorganic salts, such as an ammonium salt; and organic amine salts, such as a triethylamine salt or guanidine salt.

For cases where $R^1$ of the compound represented by general formula (I) of the present invention is a carboxy group protected by a protective group, when administered intravitally (such as in an in vivo test), the compound is easily hydrolyzed by a biochemical reaction (e.g., esterase or the like) in vivo, and thus can be converted to a pharmacologically active form in which $R^1$ is a carboxy group.

A representative method for producing the compound of the present invention is indicated below. Note that specific methods for producing each compound of the present invention are described in detail in Examples to be subsequently described.

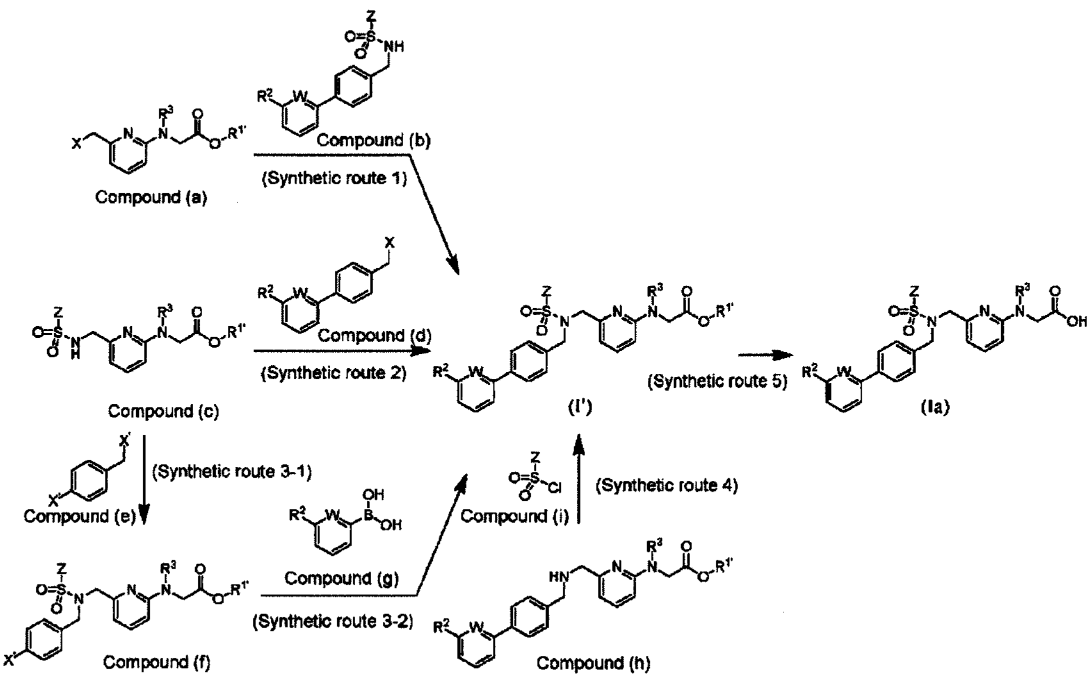

Wherein $R^2$, W, and Z are as defined in the above, $R^{1'}$ represents a protective group of the carboxy group, $R^3$ represents a tert-butoxycarbonyl group or hydrogen atom, X represents a hydroxy group, chloro group, bromo group, iodo group, methanesulfonyloxy group, benzenesulfonyloxy group, p-toluenesulfonyloxy group or trifluoromethanesulfonyloxy group, and X' represents a chloro group, bromo group, or iodo group.

The compound represented by general formula (I) of the present invention can be obtained by any method of synthetic routes 1 to 5, as a compound (Ia), in which $R^3$ is a hydrogen atom, for cases where $R^1$ is a carboxy group, or as a compound (I'), in which $R^3$ is a hydrogen atom, for cases where $R^1$ is a carboxy group protected by a protective group.

Synthetic route 1

When X is a hydroxy group in the compound (a), the compound (I') can be obtained by reacting the compound (a) with the compound (b) in an inert organic solvent and in the presence of an azo compound-based condensing agent and a phosphine reagent.

The inert organic solvent used is not particularly limited as long as it does not inhibit the reaction and dissolves the raw materials to a certain degree, and examples thereof include aromatic hydrocarbons, such as benzene, toluene or xylene; ethers, such as diethyl ether, tetrahydrofuran, 1,4-dioxane or 1,2-dimethoxyethane; amides, such as N,N-dimethylformamide, N,N-dimethylacetamide or N-methylpyrrolidone; nitriles, such as acetonitrile or propionitrile; esters, such as methyl acetate, ethyl acetate, or isopropyl acetate; and arbitrary mixed solvents thereof, and preferably tetrahydrofuran, N,N-dimethylformamide and acetonitrile, or a mixed solvent thereof.

Examples of the azo compound-based condensing agent used include diethyl azodicarboxylate (DEAD), diisopropyl azodicarboxylate (DIAD), N,N,N',N'-tetraisopropylazodicarboxamide (TIPA), 1,1'-(azodicarbonyl)dipiperidine (ADDP), N,N,N',N'-tetramethylazodicarboxamide (TMAD) or 1,6-dimethyl-1,5,7-hexahydro-1,4,6,7-tetrazocine-2,5-dione (DHTD), and preferably diethylazodicarboxylate (DEAD) or N,N,N',N'-tetramethylazodicarboxamide (TMAD). A molar amount of the azo compound-based condensing agent used is typically 0.9- to 10-fold, and preferably 1- to 5-fold, based on 1 mole of the compound (b).

Examples of the phosphine reagent used include trimethylphosphine, triethylphosphine, tri-n-butylphosphine or triphenylphosphine, and preferably tri-n-butylphosphine or triphenylphosphine. A molar amount of the phosphine compound used is typically 0.9- to 10-fold, and preferably 1- to 5-fold, based on 1 mole of the compound (b).

A molar amount of the compound (a) used is typically 0.8- to 2-fold, and preferably 0.9- to 1.5-fold, based on 1 mole of the compound (b).

Although varying depending on the types and amounts used of raw materials, solvents and the like, the reaction temperature is typically -20°C to 100°C and preferably -5°C to 50°C.

Although varying depending on the reaction temperature and the like, the reaction time is typically 30 minutes to 48 hours and preferably 1 hour to 24 hours.

When X in the compound (a) is a chloro group, bromo group, iodo group, methanesulfonyloxy group, benzenesulfonyloxy group, p-toluenesulfonyloxy group or trifluoromethanesulfonyloxy group, the compound (I') can be obtained by reacting the compound (a) with the compound (b) in an inert organic solvent and in the presence of a base.

The inert solvent used is not particularly limited as long as it does not inhibit the reaction and dissolves the raw materials to a certain degree, and examples of the inert solvent include ethers, such as tetrahydrofuran, 1,4-dioxane or 1,2-dimethoxyethane; halogenated aliphatic hydrocarbons, such as methylene chloride, chloroform or 1,2-dichloroethane; nitriles, such as acetonitrile or propionitrile; esters, such as methyl formate, ethyl formate, methyl acetate or ethyl acetate; aromatic hydrocarbons, such as benzene or toluene; amides, such as N,N-dimethylformamide, N,N-dimethylacetamide or N-methylpyrrolidone; sulfoxides, such as dimethyl sulfoxide; and arbitrary mixed solvents thereof, and preferably tetrahydrofuran, N,N-dimethylformamide, methylene chloride or 1,2-dichloroethane.

Examples of the base used include alkali metal hydrides, such as sodium hydride or potassium hydride; alkali metal amides, such as lithium amide, sodium amide, lithium diisopropylamide or lithium bis(trimethylsilyl)amide; alkali metal alkoxides, such as sodium methoxide, sodium ethoxide, sodium tert-butoxide or potassium tert-butoxide; alkali metal carbonates, such as sodium carbonate or potassium carbonate; and amines, such as triethylamine, tributylamine, diisopropylethylamine, pyridine, picoline, 2,6-lutidine or 4-dimethylaminopyridine, and preferably sodium hydride, potassium carbonate, triethylamine or diisopropylethylamine. However, when the inert solvent used is an ester, nitrile or halogenated aliphatic hydrocarbon, the base is preferably triethylamine or diisopropylethylamine.

A molar amount of the base used is typically 1- to 5-fold, and preferably 1- to 2.5-fold, based on 1 mole of the compound (b).

A molar amount of the compound (a) used is typically 0.5- to 3-fold, and preferably 0.5- to 1.5-fold, based on 1 mole of the compound (b).

Although varying depending on the types and amounts used of raw materials, solvents, and the like, the reaction temperature is typically -80°C to 100°C and preferably 0°C to 80°C.

Although varying depending on the reaction temperature and the like, the reaction time is typically 10 minutes

to 48 hours and preferably 1 hour to 24 hours.

Synthetic route 2

When X is a hydroxy group in the compound (d), the compound (I') can be obtained by reacting the compound (c) with the compound (d) in an inert organic solvent and in the presence of an azo compound-based condensing agent and phosphine reagent. This step is performed in accordance with the case where X in the compound (a) is a hydroxy group in "Synthetic route 1" described above except for using the compound (d) in place of the compound (a) and using the compound (c) in place of the compound (b).

When X in the compound (d) is a chloro group, bromo group, iodo group, methanesulfonyloxy group, benzenesulfonyloxy group, p-toluenesulfonyloxy group or trifluoromethanesulfonyloxy group, the compound (I') can be obtained by reacting the compound (c) with the compound (d) in an inert organic solvent and in the presence of a base. This step is performed in accordance with the case where X in the compound (a) is a chloro group, bromo group, iodo group, methanesulfonyloxy group, benzenesulfonyloxy group, p-toluenesulfonyloxy group or trifluoromethanesulfonyloxy group in "Synthetic route 1" described above except for using the compound (d) in place of the compound (a) and using the compound (c) in place of the compound (b).

Synthetic route 3

Synthetic route 3-1 is a step for obtaining the compound (f) by reacting the compound (c) with the compound (e) in an inert organic solvent and in the presence of a base. This step is performed in accordance with the case where X in the compound (a) is a chloro group, bromo group, iodo group, methanesulfonyloxy group, benzenesulfonyloxy group, p-toluenesulfonyloxy group or trifluoromethanesulfonyloxy group in "Synthetic route 1" described above except for using the compound (e) in place of the compound (a) and using the compound (c) in place of the compound (b).

In Synthetic route 3-2, the compound (I') can be obtained by reacting the compound (f) obtained in Synthetic route 3-1 with the compound (g) in an inert solvent in an inert gas atmosphere and in the presence of a palladium catalyst and either a base or a fluoride.

The inert solvent used is not particularly limited as long as it does not inhibit the reaction and dissolves the raw materials, catalyst, and base (or fluoride) to a certain degree, and examples thereof include aromatic hydrocarbons, such as benzene or toluene; ethers, such as tetrahydrofuran, 1,2-dimethoxyethane or 1,4-dioxane; alcohols, such as methanol, ethanol, propanol or isopropanol; esters, such as methyl acetate or ethyl acetate; amides, such as N,N-dimethylformamide, N,N-dimethylacetamide or N-methylpyrrolidone; sulfoxides, such as dimethyl sulfoxide; nitriles, such as acetonitrile; water; and arbitrary mixed solvents thereof, and preferably toluene, toluene-ethanol-water mixed solvent or toluene-water mixed solvent.

Examples of the inert gas used include nitrogen, helium and argon.

Examples of the palladium catalyst used include metal palladiums, such as palladium-activated carbon or palladium black; organopalladium complexes, such as tetrakis(triphenylphosphine)palladium, bis(triphenylphosphine)palladium chloride, 1,1'-bis(diphenylphosphino)ferrocene palladium chloride or tris(dibenzylideneacetone)dipalladium; and, palladium salts, such as palladium chloride or palladium acetate, and preferably tetrakis(triphenylphosphine)palladium or palladium acetate. A molar amount of palladium used as the catalyst is typically 0.0001- to 1-fold, and preferably 0.005- to 0.3-fold, based on 1 mole of the compound (f).

When tris(dibenzylideneacetone)dipalladium, palladium chloride or palladium acetate is used as the catalyst, it is preferably used in the presence of an organophosphine compound. Examples of the organophosphine compound used include tri-n-butylphosphine, tri-tert-butylphosphine, tricyclohexylphosphine, butyl di-1-adamantylphosphine, triphenylphosphine, tri(o-tolyl)phosphine, 2-dicyclohexylphosphino-2',6'-dimethoxybiphenyl, 1,1'-bis(diphenylphosphino)ferrocene or 1,2,3,4,5-pentaphenyl-1'-(di-tert-butylphosphino)ferrocene, and preferably tricyclohexylphosphine, butyl di-1-adamantylphosphine, triphenylphosphine or 2-dicyclohexylphosphino-2',6'-dimethoxyhiphenyl. A molar amount of the organophosphine compound used is typically 1- to 5-fold, and preferably 1.5- to 2.5-fold, based on 1 mole of the palladium.

Examples of the base or fluoride used include alkali metal acetates, such as sodium acetate or potassium acetate; alkali metal carbonates, such as sodium carbonate, potassium carbonate or cesium carbonate; alkali metal phosphates, such as trisodium phosphate or tripotassium phosphate; alkali metal hydroxides, such as lithium hydroxide, sodium hydroxide or potassium hydroxide; quaternary ammonium hydroxides, such as tetramethylammonium hydroxide, tetraethylammonium hydroxide or tetrabutylammonium hydroxide; and fluorides, such as cesium fluoride, tetramethylammonium fluoride, tetraethylammonium fluoride or tetrabutylammonium fluoride, and preferably sodium carbonate or tripotassium phosphate. A molar amount of the base or fluoride used is typically 1- to 10-fold, and preferably 1.5- to 5-fold, based on 1 mole of the compound (f).

A molar amount of the compound (g) used is typically 1- to 3-fold, and preferably 1- to 2-fold, based on 1 mole

of the compound (g).

Although varying depending on the types and amounts used of raw materials, solvents and the like, the reaction temperature is typically 0°C to 200°C and preferably 50°C to 150°C.

Although varying depending on the reaction temperature and the like, the reaction time is typically 10 minutes to 120 hours and preferably 1 hour to 48 hours.

Synthetic route 4

The compound (I') can be obtained by reacting the compound (h) with the compound (i) in an inert organic solvent and in the presence or absence of (preferably in the presence of) a base.

The inert organic solvent used is not particularly limited as long as it does not inhibit the reaction and dissolves the raw materials to a certain degree, and examples thereof include aromatic hydrocarbons, such as benzene, toluene or xylene; halogenated aliphatic hydrocarbons, such as methylene chloride, chloroform or 1,2-dichloroethane; ethers, such as 1,4-dioxane, tetrahydrofuran, diethyl ether or 1,2-dimethoxyethane; amides, such as N,N-dimethylformamide, N,N-dimethylacetamide or N-methylpyrrolidone; nitriles, such as acetonitrile or propionitrile, and arbitrary mixed solvents thereof, and preferably methylene chloride, 1,2-dichloroethane, N,N-dimethylformamide, acetonitrile or a mixed solvent thereof.

Examples of the base used include organic bases such as triethylamine or diisopropylethylamine; and inorganic bases such as sodium bicarbonate, potassium bicarbonate, sodium carbonate or potassium carbonate, and preferably triethylamine or diisopropylethylamine. A molar amount of the base used is typically 0.9- to 20-fold, and preferably 1- to 10-fold, based on 1 mole of the compound (i).

A molar amount of the compound (h) used is typically 0.7- to 5-fold, and preferably 0.8- to 1.5-fold, based on 1 mole of the compound (h).

Although varying depending on the types and amounts used of raw materials, solvents and the like, the reaction temperature is typically -20°C to 100°C and preferably -5°C to 50°C.

Although varying depending on the reaction temperature and the like, the reaction time is typically 1 minute to 36 hours and preferably 1 hour to 18 hours.

Synthetic route 5

When $R^3$ in the compound (I') is a tert-butoxycarbonyl group, the compound represented by general formula (I), in which $R^1$ is a carboxy group protected by the ester-type protective group, can be obtained by deprotecting the compound (I') by acid treatment. However, when $R^{1'}$ is a tert-butyl group and $R^3$ is a tert-butoxycarbonyl group in the compound (I'), the compound represented by general formula (I), in which $R^1$ is a carboxy group, can be obtained by deprotecting by acid treatment with hydrochloric acid, trifluoroacetic acid and the like. Similarly, when $R^3$ in the compound (I') is a hydrogen atom, the compound of general formula (I), in which $R^1$ is a carboxy group, can be obtained by suitably deprotecting the compound (I') by alkaline hydrolysis and the like.

For the substituent $R^2$, a desired substituent may be introduced at the beginning, or a desired substituent may be introduced, after having its basic structure produced according to the method described above, using a commonly used synthesizing method including oxidation, reduction, alkylation, esterification, amidation, dehydration reaction, deprotection reaction, hydrolysis, coupling reaction, cyclization reaction and/or a combination thereof.

The starting compound of the compound of the present invention is commercially available or can be produced according to a production method that is publicly known by those skilled in the art. The methods for producing the starting compound and an intermediate compound of the compound of the present invention are described in detail in Reference Examples to be subsequently described.

The target compound formed in each of the reactions can be obtained from the reaction mixture in accordance with conventional methods. For example, after suitably neutralizing the reaction mixture, or removing insolubles by filtration in the case such insolubles are present, an organic solvent such as ethyl acetate that is immiscible with water is added followed by washing with water, separating the organic layer containing the target compound, drying using a drying agent such as anhydrous magnesium sulfate or anhydrous sodium sulfate and then distilling off the solvent to obtain the target compound.

If necessary, the obtained target compound can be separated and purified by suitably combining conventional methods, such as recrystallization, re-precipitation or typical methods that have been commonly used for separation and purification of organic compounds (e.g., adsorption column chromatography methods using silica gel, alumina, or the like as a carrier, ion-exchange chromatography methods or normal phase/reversed phase column chromatography methods using silica gel or alkylated silica gel (and preferably high performance liquid chromatography)).

The compound represented by general formula (I), or the pharmacologically acceptable salt thereof, of the present invention can be present as a hydrate or a solvate.

A pharmaceutical composition containing the substituted biaryl compound represented by general formula (I), or the pharmacologically acceptable salt thereof, as an active ingredient thereof can be administered alone (as bulk powder), or the compound can be administered orally or parenterally (intravenous administration, intramuscular administration, intraperitoneal administration, dermal administration, transnasal administration, intrabronchial administration, pulmonary administration, intracutaneous administration, subcutaneous administration and the like) in the dosage form, such as a tablet, capsule, powder, syrup, granule, fine granule, pill, suspension, emulsion, percutaneous absorption agent, suppository, ointment, lotion, aerosol, powder inhalation agent or injection, that is produced by mixing with suitable pharmacologically acceptable excipients, diluents and the like.

These dosage forms are prepared by commonly known methods using additives such as excipients, lubricants, binders, disintegrators, emulsifiers, stabilizers, corrigents or diluents.

Examples of the excipients include organic excipients and inorganic excipients. Examples of the organic excipients include sugar derivatives, such as lactose, sucrose, glucose, mannitol or sorbitol; starch derivatives, such as corn starch, potato starch, α-starch or dextrin; cellulose derivatives, such as crystalline cellulose; gum arabic; dextran; and pullulan. Examples of the inorganic excipients include light anhydrous silicic acid and sulfates such as calcium sulfate.

Examples of the lubricants include stearic acid; stearic acid metal salts, such as calcium stearate or magnesium stearate; talc; colloidal silica; waxes, such as beeswax or spermaceti wax; boric acid; adipic acid; sulfates, such as sodium sulfate; glycol; fumaric acid; sodium benzoate; D,L-leucine; sodium lauryl sulfate; silicic acids, such as silicic acid anhydride or silicic acid hydrate; and starch derivatives described above for the excipients.

Examples of the binders include hydroxypropyl cellulose, hydroxypropyl methyl cellulose, polyvinylpyrrolidone, Macrogol and compounds described above for the excipients.

Examples of the disintegrators include cellulose derivatives, such as lowly substituted hydroxypropyl cellulose, carboxymethyl cellulose, calcium carboxymethyl cellulose or internally crosslinked calcium carboxymethyl cellulose; crosslinked polyvinylpyrrolidone; and chemically modified starch or cellulose derivatives, such as carboxymethyl starch or sodium carboxymethyl starch.

Examples of the emulsifiers include colloidal clays, such as bentonite or Veegum; anionic surfactants, such as sodium lauryl sulfate; cationic surfactants, such as benzalkonium chloride; and nonionic surfactants, such as polyoxyethylene alkyl ether, polyoxyethylene sorbitan fatty acid ester or sucrose fatty acid ester.

Examples of the stabilizers include para-hydroxybenzoates, such as methylparaben or propylparaben; alcohols, such as chlorobutanol, benzyl alcohol, or phenylethyl alcohol; benzalkonium chloride; phenols, such as phenol or cresol; thimerosal; acetic anhydride; and sorbic acid.

Examples of corrigents include sweeteners, such as saccharin sodium or aspartame; acidulants, such as citric acid, malic acid or tartaric acid; and flavorings, such as menthol, lemon extract or orange extract.

Examples of diluents include compounds commonly used as diluents, such as lactose, mannitol, glucose, sucrose, calcium sulfate, hydroxypropyl cellulose, microcrystalline cellulose, water, ethanol, polyethylene glycol, propylene glycol, glycerol, starch, polyvinylpyrrolidone or mixtures thereof.

In addition, suitable additives can be used depending on the dosage form. For example, when the compound represented by general formula (I), or the pharmacologically acceptable salt thereof, of the present invention is formed into an aerosol for transnasal administration or intrabronchial administration, chlorofluorocarbons (CFCs), such as dichlorodifluoromethane, trichlorofluoromethane or dichlorotetrafluoroethane, or carbon dioxide can be used as a propellant.

Although the dosage of the active ingredient of the pharmaceutical composition of the present invention can be varied depending on the conditions such as symptoms, age or weight of the patient, the dosage for an adult in the case of a single oral administration has a lower limit of 0.001 mg/kg (preferably 0.01 mg/kg) and an upper limit of 20 mg/kg (preferably 10 mg/kg), while the dosage for an adult in the case of a single parenteral administration has a lower limit of 0.0001 mg/kg (preferably 0.0005 mg/kg) and an upper limit of 10 mg/kg (preferably 5 mg/kg), and these can be administered 1 to 6 times per day corresponding to symptoms.

## EXAMPLES

Although the following provides a more detailed explanation of the present invention through Examples, Reference Examples, Comparative Examples, Test Examples and Preparation Examples, the present invention is not limited thereto.

[Example 1]

<u>Ethyl (6-{[3'-(1-propenyl)biphenyl-4-ylmethyl](pyridin-2-ylsulfonyl)aminomethyl}pyridin-2-ylamino)acetate</u>

320 mg (0.913 mmol) of ethyl {6-[(pyridin-2-ylsulfonyl)aminomethyl]pyridin-2-ylamino}acetate obtained according to the same method as Reference Example 1-(g), 570 μL (2.31 mmol) of tri-n-butylphosphine and 236 mg (1.37

mmol) of N,N,N',N'-tetramethylazodicarboxamide were added to 9.4 mL solution of tetrahydrofuran containing 205 mg (0.931 mol) of 3'-(1-propenyl)biphenyl-4-ylmethanol obtained in Reference Example 3-(b) followed by stirring for 5 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 2:3 (V/V)) and the fractions containing the target product were concentrated under reduced pressure to obtain 510 mg of the title compound in the form of a slightly yellow oil (quantitative).
Mass spectrum (FAB, m/z): 557 ($M^+$+1).
$^1$H-NMR spectrum ($CDCl_3$, δ ppm): 8.62 (ddd, J = 4.7, 1.8, 1.0 Hz, 1H), 7.83 (ddd, J = 7.8, 1.0, 1.0 Hz, 1H), 7.75 (ddd, J = 7.8, 7.6, 1.8 Hz, 1H), 7.52-7.43 (m, 3H), 7.41-7.30 (m, 6H), 7.27-7.20 (m, 1H), 6.51 (d, J = 7.3 Hz, 1H), 6.50-6.42 (m, 1H), 6.38-6.26 (m, 1H), 6.23 (d, J = 8.3 Hz, 1H), 4.80 (s, 2H), 4.70 (t, J = 5.4 Hz, 0.9H), 4.42 (s, 2H), 4.22 (q, J = 7.1 Hz, 2H), 3.96 (d, J = 5.4 Hz, 2H), 1.91 (dd, J = 6.3, 1.5 Hz, 3H), 1.28 (t, J = 7.1 Hz, 3H).

[Example 2]

(6-{[3'-(1-Propenyl)biphenyl-4-ylmethyl](pyridin-2-ylsulfonyl)aminomethyl}-pyridin-2-ylamino)acetic acid

1.98 mL (1.98 mmol) of 1 mol/L aqueous sodium hydroxide solution was added to 2.0 mL solution of ethanol containing 220 mg (0.395 mmol) of the ethyl (6-{[3'-(1-propenyl)biphenyl-4-ylmethyl](pyridin-2-ylsulfonyl)aminomethyl} pyridin-2-ylamino)acetate obtained in Example 1 followed by stirring for 2.5 hours at room temperature. After completion of the reaction, water was added to the reaction solution followed by adjusting the pH to 4.5 with 1 mol/L hydrochloric acid. The precipitated solid was collected by filtration and then dried under reduced pressure to obtain 146 mg of the title compound in the form of a white solid (yield: 70%).
mass spectrum (FAB, m/z): 529 ($M^+$+1).
$^1$H-NMR spectrum (DMSO-$d_6$, δ ppm): 8.64 (ddd, J = 4.8, 1.7, 0.9 Hz, 1H), 7.95 (ddd, J = 7.7, 7.7, 1.7 Hz, 1H), 7.80 (ddd, J = 7.7, 1.0, 0.9 Hz, 1H), 7.61-7.56 (m, 4H), 7.48-7.44 (m, 1H), 7.39-7.37 (m, 2H), 7.35-7.32 (m, 2H), 7.19 (dd, J = 8.3, 7.2 Hz, 1H), 6.61 (brs, 0.8H), 6.52-6.47 (m, 1H), 6.44-6.37 (m, 1H), 6.33 (d, J = 8.3 Hz, 1H), 6.28 (d, J = 7.2 Hz, 1H), 4.74 (s, 2H), 4.24 (s, 2H), 3.76 (d, J = 4.0 Hz, 2H), 1.87 (dd, J = 6.2, 1.5 Hz, 3H).

[Example 3]

Ethyl (6-{[3'-1-propynyl)biphenyl-4-ylmethyl](pyridin-2-ylsulfonyl)aminomethyl}pyridin-2-ylamino)acetate

315 mg (0.900 mmol) of ethyl {6-[(pyridin-2-ylsulfonyl)aminomethyl]pyridin-2-ylamino}acetate obtained according to the same method as Reference Example 1-(g), 450 μL (1.82 mmol) of tri-n-butylphosphine and 310 mg (1.80 mmol) of N,N,N',N'-tetramethylazodicarboxamide were added to 4.0 mL solution of tetrahydrofuran containing 200 mg (0.900 mol) of 3'-(1-propynyl)biphenyl-4-ylmethanol obtained in Reference Example 4-(b) followed by stirring for 3 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 3:2→2:3 (V/V)) and the fractions containing the target product were concentrated under reduced pressure to obtain 483 mg of the title compound in the form of a white foam (yield: 97%).
mass spectrum (FAB, m/z): 555 ($M^+$+1).
$^1$H-NMR spectrum ($CDCl_3$, δ ppm): 8.62 (ddd, J = 4.6, 1.7, 1.0 Hz, 1H), 7.83 (ddd, J = 7.7, 1.3, 1.0 Hz, 1H), 7.75 (ddd, J = 7.7, 7.7, 1.7 Hz, 1H), 7.59-7.58 (m, 1H), 7.47-7.43 (m, 3H), 7.41-7.31 (m, 5H), 7.23 (dd, J = 8.2, 7.1 Hz, 1H), 6.51 (d, J = 7.1 Hz, 1H), 6.23 (d, J = 8.2 Hz, 1H), 4.79 (s, 2H), 4.70 (t, J = 5.4 Hz, 1H), 4.42 (s, 2H), 4.22 (q, J = 7.1 Hz, 2H), 3.96 (d, J = 5.4 Hz, 2H), 2.08 (s, 3H), 1.28 (t, J = 7.1 Hz, 3H).

[Example 4]

(6-{[3'-(1-Propynyl)biphenyl-4-ylmethyl](pyridin-2-ylsulfonyl)aminomethyl}-pyridin-2-ylamino)acetic acid

3.43 mL (3.43 mmol) of 1 mol/L aqueous sodium hydroxide solution was added to 3.0 mL solution of ethanol containing 476 mg (0.858 mmol) of the ethyl (6- {[3'-(1-propynyl)biphenyl-4-ylmethyl](pyridin-2-ylsulfonyl)aminomethyl} pyridin-2-ylamino)acetate obtained in Example 3 followed by stirring for 5 hours at room temperature. After completion of the reaction, water was added to the reaction solution and the pH was adjusted to 4.5 with 1 mol/L hydrochloric acid

followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: methylene chloride:methanol =15:1→10:1 (V/V)) and the fractions containing the target product were concentrated under reduced pressure to obtain 444 mg of the title compound in the form of a white foam (yield: 98%).
mass spectrum (FAB, m/z): 527 ($M^+$+1).
$^1$H-NMR spectrum (DMSO-$d_6$, δ ppm): 12.42 (brs, 0.6H), 8.64 (ddd, J = 4.7, 1.8, 1.0 Hz, 1H), 7.95 (ddd, J = 7.8, 7.7, 1.8 Hz, 1H), 7.80 (ddd, J = 7.8, 1.0, 1.0 Hz, 1H), 7.63-7.56 (m, 5H), 7.43 (dd, J = 7.9, 7.9 Hz, 1H), 7.37 (ddd, J = 7.9, 1.7, 0.9 Hz, 1H), 7.35-7.32 (m, 2H), 7.19 (dd, J = 8.4, 7.0 Hz, 1H), 6.75 (t, J = 5.9 Hz, 1H), 6.34 (d, J = 8.4 Hz, 1H), 6.28 (d, J = 7.0 Hz, 1H), 4.74 (s, 2H), 4.24 (s, 2H), 3.82 (d, J = 5.9 Hz, 2H), 2.07 (s, 3H).

[Example 5]

Ethvl (6-{[3'-(1-propynyl)biphenyl-4-ylmethyl](pyridin-3-ylsulfonyl)aminomethyl}pyridin-2-ylamino)acetate

280 mg (0.800 mmol) of ethyl {6-[(pyridin-3-ylsulfonyl)aminomethyl]pyridin-2-ylamino}acetate obtained according to the same method as Reference Example 2-(b), 395 μL (1.60 mmol) of tri-n-butylphosphine and 276 mg (1.60 mmol) of N,N,N',N'-tetramethylazodicarboxamide were added to 4.0 mL solution of tetrahydrofuran containing 178 mg (0.800 mol) of 3'-(1-propynyl)biphenyl-4-ylmethanol obtained in Reference Example 4-(b) followed by stirring for 3 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 3:7→0:1 (V/V)) and the fractions containing the target product were concentrated under reduced pressure to obtain 400 mg of the title compound in the form of a slightly yellow oil (yield: 90%).
mass spectrum (ESI$^+$, m/z): 555 ($M^+$+1).
$^1$H-NMR spectrum ($CDCl_3$, δ ppm): 8.97 (dd, J = 2.3, 0.7 Hz, 1H), 8.69 (dd, J = 4.9, 1.7 Hz, 1H), 7.92 (ddd, J = 8.0, 2.3, 1.7 Hz, 1H), 7.61-7.60 (m, 1H), 7.52-7.49 (m, 2H), 7.48-7.46 (m, 1H), 7.38-7.35 (m, 4H), 7.32-7.27 (m, 2H), 6.46 (d, J = 7.0 Hz, 1H), 6.28 (d, J = 8.2 Hz, 1H), 4.74 (t, J = 5.4 Hz, 1H), 4.66 (s, 2H), 4.34 (s, 2H), 4.22 (q, J = 7.2 Hz, 2H), 3.87 (d, J = 5.4 Hz, 2H), 2.08 (s, 3H), 1.29 (t, J = 7.2 Hz, 3H).

[Example 6]

(6-{[3'-(1-Propynyl)biphenyl-4-ylmethyll(pyridin-3-ylsulfonyl)aminomethyl}-pyridin-2-ylamino)acetic acid

3.0 mL (3.00 mmol) of 1 mol/L aqueous sodium hydroxide solution was added to 3.0 mL solution of ethanol containing 395 mg (0.712 mmol) of the ethyl (6-{[3'-(1-propynyl)biphenyl-4-ylmethyl](pyridin-3-ylsulfonyl)aminomethyl} pyridin-2-ylamino)acetate obtained in Example 5 followed by stirring for 16 hours at room temperature. After completion of the reaction, water was added to the reaction solution and the pH was adjusted to 4.5 with 1 mol/L hydrochloric acid followed by extracting with ethyl acetate. The organic layer was washed with saturated aqueous sodium chloride solution and dried with anhydrous sodium sulfate followed by concentration under reduced pressure. 10 mL of tert-butyl methyl ether and 0.5 mL of methanol were added to the residue and the solid that precipitated following sonification was collected by filtration and then dried under reduced pressure to obtain 340 mg of the title compound in the form of a white solid (yield: 91%).
mass spectrum (ESI$^+$, m/z): 527 ($M^+$+1).
$^1$H-NMR spectrum (DMSO-$d_6$, δ ppm): 12.42 (brs, 0.6H), 8.83 (dd, J = 2.4, 0.6 Hz, 1H), 8.72 (dd, J = 4.8, 1.6 Hz, 1H), 8.02 (ddd, J = 8.1, 2.4, 1.6 Hz, 1H), 7.65-7.61 (m, 4H), 7.47 (ddd, J = 8.1, 4.8, 0.6 Hz, 1H), 7.44 (dd, J = 7.9,7.9 Hz, 1H), 7.39-7.36 (m, 3H), 7.24 (dd, J = 8.3, 7.1 Hz, 1H), 6.78 (t, J = 5.9 Hz, 1H), 6.37 (d, J = 8.3 Hz, 1H), 6.33 (d, J = 7.1 Hz, 1H), 4.71 (s, 2H), 4.21 (s, 2H), 3.71 (d, J = 5.9 Hz, 2H), 2.07 (s, 3H).

[Example 7]

{6-[(3'-Ethoxybiphenyl-4-ylmethyl)(pyridin-2-ylsulfonyl)aminomethyl]-pyridin-2-ylamino}acetic acid

7-(a): tert-Butyl (tert-butoxycarbonyl{6-[(3'-ethoxybiphenyl-4-ylmethyl) (pyridin-2-ylsulfonyl)-aminomethyl]pyridin-2-ylamino}acetate

422 mg (0.880 mmol) of tert-butyl {tert-butoxycarbonyl{6-[(pyridin-2-ylsulfonyl)aminomethyl]pyridin-2-ylamino}acetate obtained according to the same method as Reference Example 1-(f), 395 μL (1.60 mmol) of tri-n-butylphosphine and 276 mg (1.60 mmol) of N,N,N',N'-tetramethylazodicarboxamide were added to 4.0 mL solution of tetrahydrofuran containing 183 mg (0.800 mol) of 3'-ethoxybiphenyl-4-ylmethanol obtained in Reference Example 5 followed by stirring for 3 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: toluene:ethyl acetate = 8:1→6:1 (V/V)) and the fractions containing the target product were concentrated under reduced pressure to obtain 537 mg of the title compound in the form of a white foam (yield: 98%).
mass spectrum (FAB, m/z): 689 ($M^{+}$+1).
$^{1}$H-NMR spectrum ($CDCl_3$, δ ppm): 8.60 (ddd, J = 4.6, 1.8, 1.0 Hz, 1H), 7.82 (ddd, J = 7.7, 1.0, 1.0 Hz, 1H), 7.77 (ddd, J = 7.7, 7.6, 1.8 Hz, 1H), 7.65 (d, J = 8.3 Hz, 1H), 7.48-7.26 (m, 7H), 7.11 (ddd, J = 7.9, 1.7, 0.9 Hz, 1H), 7.07 (dd, J = 2.3, 1.7 Hz, 1H), 6.91 (d, J = 7.3 Hz, 1H), 6.88 (ddd, J = 7.9, 2.3, 0.9 Hz, 1H), 4.74 (s, 2H), 4.51 (s, 2H), 4.46 (s, 2H), 4.10 (q, J = 6.9 Hz, 2H), 1.52 (s, 9H), 1.45 (t, J = 6.9 Hz, 3H), 1.42 (s, 9H).

7-(b): <u>{6-[(3'- Ethoxybiphenyl-4-ylmethyl)(pyridin-2-ylsulfonyl)aminomethyl]-pyridin-2-ylamino}acetic acid</u>

3.2 mL (19.2 mmol) of 6 mol/L hydrochloric acid and 0.8 mL of water were added to 4.0 mL solution of 1,4-dioxane containing 525 mg (0.762 mmol) of the tert-butyl (tert-butoxycarbonyl {6-[(3'-ethoxybiphenyl-4-ylmethyl) (pyridin-2-ylsulfonyl)aminomethyl]pyridin-2-ylamino acetate obtain in Example 7-(a) followed by stirring for 3 hours at 70°C. After completion of the reaction, the reaction solution was concentrated under reduced pressure followed by addition of water, adjusting the pH to 4.4 with 1 mol/L aqueous sodium hydroxide solution and extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: methylene chloride:methanol = 15:1→10:1 (V/V)) and the fractions containing the target product were concentrated under reduced pressure to obtain 369 mg of the title compound in the form of a white foam (yield: 91%).
mass spectrum (FAB, m/z): 533 ($M^{+}$+1).
$^{1}$H-NMR spectrum (DMSO-$d_6$, δ ppm): 12.41 (brs, 0.4H), 8.64 (ddd, J = 4.6, 1.8, 0.9 Hz, 1H), 7.95 (ddd, J = 7.8, 7.8, 1.8 Hz, 1H), 7.80 (ddd, J = 7.8, 1.0, 0.9 Hz, 1H), 7.59-7.56 (m, 3H), 7.36 (dd, J = 8.1, 8.1 Hz, 1H), 7.33-7.31 (m, 2H), 7.20 (dd, J = 8.2, 7.1 Hz, 1H), 7.18 (ddd, J = 8.1, 1.8, 0.8 Hz, 1H), 7.14 (dd, J = 2.3, 1.8 Hz, 1H), 6.92 (ddd, J = 8.1, 2.3,0.8 Hz, 1H), 6.75 (t, J = 5.9 Hz, 1H), 6.34 (d, J = 8.2 Hz, 1H), 6.28 (d, J = 7.1 Hz, 1H), 4.74 (s, 2H), 4.24 (s, 2H), 4.10 (q, J = 7.0 Hz, 2H), 3.82 (d, J = 5.9 Hz, 2H), 1.35 (t, J = 7.0 Hz, 3H).

[Example 8]

<u>Hexyl {6-[(3'-ethoxybiphenyl-4-ylmethyl)(pyridin-2-ylsulfonyl)aminomethy]-pyridin-2-ylamino}acetate</u>

305 mg (0.750 mmol) of the hexyl {6-[(pyridin-2-ylsulfonyl)aminomethyl]pyridin-2-ylamino}acetate obtained in Reference Example 6,280 μL (1.14 mmol) of tri-n-butylphosphine and 196 mg (1.14 mmol) of N,N,N',N'-tetramethylazodicarboxamide were added to 4.0 mL solution of tetrahydrofuran containing 171 mg (0.750 mol) of 3'-ethoxybiphenyl-4-ylmethanol obtained in Reference Example 5 followed by stirring for 16 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 3:2→2:3 (V/V)) and the fractions containing the target product were concentrated under reduced pressure to obtain 429 mg of the title compound in the form of a colorless oil (yield: 93%).
mass spectrum (FAB, m/z): 617 ($M^{+}$+1).
$^{1}$H-NMR spectrum ($CDCl_3$, δ ppm): 8.61 (ddd, J = 4.7, 1.7, 1.0 Hz, 1H), 7.82 (ddd, J = 7.7, 1.1, 1.0 Hz, 1H), 7.75 (ddd, J = 7.7, 7.7, 1.7 Hz, 1H), 7.47-7.45 (m, 2H), 7.38 (ddd, J = 7.7, 4.7, 1.1 Hz, 1H), 7.35-7.31 (m, 3H), 7.23 (dd, J = 8.4, 7.3 Hz, 1H), 7.12 (ddd, J = 8.1, 1.8, 1.0 Hz, 1H), 7.08 (dd, J = 2.4, 1.8 Hz, 1H), 6.88 (ddd, J = 8.1, 2.4, 1.0 Hz, 1H), 6.51 (d, J = 7.3 Hz, 1H), 6.23 (d, J = 8.4 Hz, 1H), 4.79 (s, 2H), 4.70 (t, J = 5.3 Hz, 1H), 4.42 (s, 2H), 4.15 (t, J = 6.8 Hz, 2H), 4.10 (q, J = 7.0 Hz, 2H), 3.96 (d, J = 5.3 Hz, 2H), 1.66-1.60 (m, 2H), 1.45 (t, J = 7.0 Hz, 3H), 1.34-1.25 (m, 6H), 0.87 (t, J = 7.0 Hz, 3H).

[Example 9]

{6-[(3'-Ethoxybiphenyl-4-ylmethyl)(pyridin-3-ylsulfonynaminomethyl]-pyridin-2-ylamino}acetic acid

9-(a): tert-Butyl (tert-butoxycarbonyl{6-[(3'-ethoxybiphenyl-4-ylmethyl) (pyridin-3-ylsulfonyl)aminomethyl]pyridin-2-ylamino}acetate

422 mg (0.880 mmol) of tert-butyl (tert-butoxycarbonyl{6-[(pyridin-3-ylsulfonyl)aminomethyl]pyridin-2-ylamino} acetate obtained according to the same method as Reference Example 2-(a), 395 μL (1.60 mmol) of tri-n-butylphosphine and 276 mg (1.60 mmol) of N,N,N',N'-tetramethylazodicarboxamide were added to 4.0 mL solution of tetrahydrofuran containing 183 mg (0.800 mol) of 3'-ethoxybiphenyl-4-ylmethanol obtained in Reference Example 5 followed by stirring for 3 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 7:3→1:1 (V/V)) and the fractions containing the target product were concentrated under reduced pressure to obtain 550 mg of the title compound in the form of a white foam (quantitative).
mass spectrum (FAB, m/z): 689 ($M^+$+1).
$^1$H-NMR spectrum ($CDCl_3$, δ ppm): 8.96 (dd, J = 2.4, 0.7 Hz, 1H), 8.71 (dd, J = 4.8, 1.6 Hz, 1H), 7.87 (ddd, J = 7.9, 2.4, 1.6 Hz, 1H), 7.71 (d, J = 8.5 Hz, 1H), 7.54-7.47 (m, 3H), 7.36-7.26 (m, 4H), 7.13 (ddd, J = 7.9, 1.9, 1.0 Hz, 1H), 7.08 (dd, J = 2.3, 1.9 Hz, 1H), 6.89 (ddd, J = 8.3, 2.3, 1.0 Hz, 1H), 6.87 (d, J = 7.3 Hz, 1H), 4.62 (s, 2H), 4.42 (s, 2H), 4.37 (s, 2H), 4.10 (q, J = 7.0 Hz, 2H), 1.52 (s, 9H), 1.45 (t, J = 7.0 Hz, 3H), 1.42 (s, 9H).

9-(b): {6-[(3'-Ethoxybiphenyl-4-ylmethyl)(pyridin-3-ylsulfonyl)aminomethyl]-pvridin-2-ylamino}acetic acid

3.3 mL (20 mmol) of 6 mol/L hydrochloric acid and 1.0 mL of water were added to 4.0 mL solution of 1,4-dioxane containing 540 mg (0.784 mmol) of the tert-butyl (tert-butoxycarbonyl{6-[(3'-ethoxybiphenyl-4-ylmethyl)(pyridin-3-ylsulfonyl) aminomethyl]pyridin-2-ylamino}acetate obtained in Example 9-(a) followed by stirring for 2 hours at 70°C. After completion of the reaction, the reaction solution was concentrated under reduced pressure followed by addition of water, adjusting the pH to 4.4 with 1 mol/L aqueous sodium hydroxide solution and extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: methylene chloride:methanol = 15:1→10:1 (V/V)) and the fractions containing the target product were concentrated under reduced pressure. 2 mL of ethyl acetate and 8 mL of n-hexane were added to the concentrate and the precipitated solid was collected by filtration and dried under reduced pressure to obtain 388 mg of the title compound in the form of a white solid (yield: 93%).
mass spectrum (FAB, m/z): 533 ($M^+$+1).
$^1$H-NMR spectrum (DMSO-$d_6$, δ ppm): 12.43 (brs, 0.4H), 8.83 (dd, J = 2.4, 0.7 Hz, 1H), 8.72 (dd, J = 4.8, 1.7 Hz, 1H), 8.02 (ddd, J = 8.0, 2.4, 1.7 Hz, 1H), 7.64-7.61 (m, 2H), 7.47 (ddd, J = 8.0, 4.8, 0.7 Hz, 1H), 7.38-7.34 (m, 3H), 7.24 (dd, J = 8.3, 7.2 Hz, 1H), 7.20 (ddd, J = 7.8, 1.7, 0.9 Hz, 1H), 7.16 (dd, J = 2.3, 1.7 Hz, 1H), 6.92 (ddd, J = 8.2, 2.3, 0.9 Hz, 1H), 6.78 (t, J = 5.9 Hz, 1H), 6.37 (d, J = 8.3 Hz, 1H), 6.33 (d, J = 7.2 Hz, 1H), 4.70 (s, 2H), 4.21 (s, 2H), 4.10 (q, J = 7.0 Hz, 2H), 3.71 (d, J = 5.9 Hz, 2H), 1.36 (t, J = 7.0 Hz, 3H).

[Example 10]

{6-[(Benzenesulfonyl)(3'-ethoxybiphenyl-4-ylmethyl)aminomethyl]pyridin-2-ylamino}acetic acid

10-(a): tert-Butyl ({6-r(benzensulfonyl)(3'-ethoxybiphenyl-4-ylmethyl)-aminomethyl]pyridin-2-yl}tert-butoxycarbonylamino)acetate

178 μL (1.28 mmol) of triethylamine and 98 μL (0.77 mmol) of benzenesulfonyl chloride were added to 1.8 mL solution of methylene chloride containing 350 mg (0.639 mmol) of tert-butyl (tert-butoxycarbonyl-{6-[(3'-ethoxybiphenyl-4-ylmethyl)aminomethyl]pyridin-2-yl}amino)acetate obtained in Reference Example 7(b) while cooling with ice followed by stirring for 1 hour at room temperature. After completion of the reaction, the reaction solution was subjected to silica gel column chromatography (elution solvent: n-hexane:ethyl acetate = 4:1→7:3 (V/V)) and the fractions containing the target product were concentrated under reduced pressure to obtain 392 mg of the title compound in the form of a white foam (yield: 89%).
mass spectrum (CI, m/z): 688 ($M^+$+1).

$^1H$-NMR spectrum ($CDCl_3$, δ ppm): 7.77-7.73 (m, 2H), 7.67 (d, J = 8.3 Hz, 1H), 7.56-7.41 (m, 6H), 7.33 (dd, J = 7.9, 7.7 Hz, 1H), 7.24-7.19 (m, 2H), 7.11 (ddd, J = 7.7, 1.7, 0.9 Hz, 1H), 7.07 (dd, J = 2.3, 1.7 Hz, 1H), 6.88 (ddd, J = 7.9, 2.3, 0.9 Hz, 1H), 6.86 (d, J = 7.5 Hz, 1H), 4.54 (s, 2H), 4.39 (s, 2H), 4.35 (s, 2H), 4.09 (q, J = 7.1 Hz, 2H), 1.51 (s, 9H), 1.44 (t, J = 7.1 Hz, 3H), 1.41 (s, 9H).

10-(b): {6-[(Benzenesulfonyl)(3'-ethoxybiphenyl-4-ylmethyl)aminomethyl]-pyridin-2-yl}acetic acid

5.8 mL (76 mmol) of trifluoroacetic acid was added at room temperature to 5.8 mL solution of methylene chloride containing 389 mg (0.566 mmol) of the tert-butyl ({6-[(benzensulfonyl)(3'-ethoxybiphenyl-4-ylmethyl)aminomethyl]-pyridin-2-yl}tert-butoxycarbonylamino)acetate obtained in Example 10-(a) followed by allowing to stand undisturbed for 3.5 hours. After completion of the reaction, the reaction solution was concentrated under reduced pressure followed by addition of water, adjusting the pH to 4.4 with saturated aqueous sodium bicarbonate solution and 1 mol/L hydrochloric acid, and extracting with ethyl acetate. The organic layer was washed with saturated aqueous sodium chloride solution and dried with anhydrous sodium sulfate followed by concentration under reduced pressure. 3.9 mL of diisopropyl ether was added to the concentrate and the precipitated solid was collected by filtration and dried under reduced pressure to obtain 293 mg of the title compound in the form of a white solid (yield: 97%).
mass spectrum (FAB, m/z): 532 ($M^+$+1).
$^1H$-NMR spectrum (DMSO-$d_6$, δ ppm): 12.41 (brs, 0.8H), 7.74-7.72 (m, 2H), 7.61-7.59 (m, 3H), 7.52-7.48 (m, 2H), 7.35 (dd, J = 7.8,7.8 Hz, 1H), 7.31-7.29 (m, 2H), 7.23 (dd, J = 8.4, 7.2 Hz, 1H), 7.19 (ddd, J = 7.8, 1.7, 0.9 Hz, 1H), 7.14 (dd, J = 2.3,1.7 Hz, 1H), 6.91 (ddd, J = 7.8, 2.3, 0.9 Hz, 1H), 6.76 (t, J = 5.9 Hz, 1H), 6.37 (d, J = 8.4 Hz, 1H), 6.29 (d, J = 7.2 Hz, 1H), 4.59 (s, 2H), 4.16 (s, 2H), 4.10 (q, J = 7.0 Hz, 2H), 3.77 (d, J = 5.9 Hz, 2H), 1.35 (t, J = 7.0 Hz, 3H).

[Example 11]

{6-[(3'-Ethoxybiphenyl-4-ylmethyl)(thiophen-2-ylsulfonyl)aminomethyl]-pyridin-2-ylamino}acetic acid

11-(a): tert-Butyl (tert-butoxycarbonyl16-[(3'-ethoxybiphenyl-4-ylmethyl) (thiophen-2-ylsulfonyl)aminomethyl]pyridin-2-yl} acetate

178 μL (1.28 mmol) of triethylamine and 0.3 mL solution of methylene chloride containing 141 mg (0.772 mmol) of 2-thiophenesulfonyl chloride were added to 1.8 mL solution of methylene chloride containing 350 mg (0.639 mmol) of tert-butyl (tert-butoxycarbonyl {6-[(3'-ethoxybiphenyl-4-ylmethyl)aminomethyl]-pyridin-2-yl}-amino)acetate obtained in Reference Example 7(b) while cooling with ice followed by stirring for 1.5 hours at room temperature. After completion of the reaction, the reaction solution was subjected to silica gel column chromatography (elution solvent: n-hexane:ethyl acetate = 9:1→3:2 (V/V)) and the fractions containing the target product were concentrated under reduced pressure to obtain 376 mg of the title compound in the form of a white foam (yield: 85%).
mass spectrum (CI, m/z): 694 ($M^+$+1).
$^1H$-NMR spectrum ($CDCl_3$, δ ppm): 7.70 (d, J = 8.5 Hz, 1H), 7.54-7.42 (m, 5H), 7.33 (dd, J = 8.0, 7.8 Hz, 1H), 7.27-7.23 (m, 2H), 7.12 (ddd, J = 7.7, 1.7, 0.9 Hz, 1H), 7.08 (dd, J = 2.4, 1.7 Hz, 1H), 7.02 (dd, J = 5.1, 3.7 Hz, 1H), 6.92 (d, J = 7.6 Hz, 1H), 6.88 (ddd, J = 8.0, 2.4, 0.9 Hz, 1H), 4.56 (s, 2H), 4.43 (s, 2H), 4.39 (s, 2H), 4.10 (q, J = 7.0 Hz, 2H), 1.52 (s, 9H), 1.44 (t, J = 7.0 Hz, 3H), 1.42 (s, 9H).

11-(b): (6-[(3'-Ethoxybiphenyl-4-ylmethyl)thiophene-2-ylsulfonyl)-aminomethyl]pyridin-2-yl}acetic acid

5.6 mL (73 mmol) of trifluoroacetic acid was added at room temperature to 5.6 mL solution of methylene chloride containing 374 mg (0.538 mmol) of the tert-butyl (tert-butoxycarbonyl {6-[(3'-ethoxybiphenyl-4-ylmethyl) (thiophen-2-ylsulfonyl)aminomethyl]pyridin-2-yl}acetate obtained in Example 11-(a) followed by allowing to stand undisturbed for 3.5 hours. After completion of the reaction, the reaction solution was concentrated under reduced pressure followed by addition of water, adjusting the pH to 4.4 with saturated aqueous sodium bicarbonate solution and 1 mol/L hydrochloric acid, and extracting with ethyl acetate. The organic layer was washed with saturated aqueous sodium chloride solution and dried with anhydrous sodium sulfate followed by concentration under reduced pressure. 3.7 mL of tert-butyl methyl ether was added to the concentrate and the precipitated solid was collected by filtration and dried under reduced pressure to obtain 272 mg of the title compound in the form of a white solid (yield: 94%).
mass spectrum (FAB, m/z): 538 ($M^+$+1).
$^1H$-NMR spectrum (DMSO-$d_6$, δ ppm): 12.42 (brs, 0.7H), 7.91 (dd, J = 5.1, 1.4 Hz, 1H), 7.61-7.58 (m, 2H), 7.54 (dd, J = 3.7, 1.4 Hz, 1H), 7.35 (dd, J = 7.9, 7.8 Hz, 1H), 7.34-7.31 (m, 2H), 7.27 (dd, J = 8.4, 7.2 Hz, 1H), 7.19 (ddd, J = 7.8, 1.7, 0.9 Hz, 1H), 7.15 (dd, J = 2.3, 1.7 Hz, 1H), 7.13 (dd, J = 5.1, 3.7 Hz, 1H), 6.91 (ddd, J = 7.9, 2.3, 0.9 Hz, 1H), 6.79 (t, J = 5.8 Hz, 1H), 6.41 (d, J = 8.4 Hz, 1H), 6.35 (d, J = 7.2 Hz, 1H), 4.58 (s, 2H), 4.17 (s, 2H), 4.10 (q, J = 7.0 Hz, 2H),

3.83 (d, J = 5.8 Hz, 2H), 1.35 (t, J = 7.0 Hz, 3H).

[Example 12]

(6-{[4-(6-Ethoxypyridin-2-yl)benzyl](pyridin-2-ylsulfonyl)aminomethyl}-pyridin-2-ylamino)acetic acid

12-(a): tert-Butyl [tert-butoxycarbonyl(6-{[4-(6-ethoxypyridin-2-yl)benzyl] (pyridin-2-ylsulfonyl)aminomethyl}pyridin-2-yl)amino]acetate

560 mg (1.17 mmol) of tert-butyl (tert-butoxycarbonyl{6-[(pyridin-2-ylsulfonyl)aminomethyl]pyridin-2-yl}amino)acetate obtained according to the same method as Reference Example 1-(f), 724 μL (2.90 mmol) of tri-n-butylphosphine and 300 mg (1.74 mmol) of N,N,N',N'-tetramethylazodicarboxamide were added to 11 mL solution of tetrahydrofuran containing 267 mg (1.16 mol) of 4-(6-ethoxypyridin-2-yl)phenyl methanol obtained in Reference Example 8 followed by stirring for 1.5 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 3:1 (V/V)) and the fractions containing the target product were concentrated under reduced pressure to obtain 606 mg of the title compound in the form of a white foam (yield: 76%).
mass spectrum (CI, m/z): 690 ($M^+$+1).
$^1$H-NMR spectrum ($CDCl_3$, δ ppm): 8.60 (ddd, J = 4.7, 1.8, 1.1 Hz, 1H), 7.92-7.88 (m, 2H), 7.82 (ddd, J = 7.7, 1.3, 1.1 Hz, 1H), 7.76 (ddd, J = 7.7, 7.5, 1.8 Hz, 1H), 7.65 (d, J = 8.5 Hz, 1H), 7.61 (dd, J = 8.2, 7.5 Hz, 1H), 7.45 (dd, J = 8.3, 7.5 Hz, 1H), 7.38 (ddd, J = 7.5, 4.7, 1.3 Hz, 1H), 7.34-7.30 (m, 2H), 7.28 (dd, J = 7.5, 0.6 Hz, 1H), 6.90 (d, J = 7.5 Hz, 1H), 6.66 (dd, J = 8.3, 0.6 Hz, 1H), 4.76 (s, 2H), 4.49 (s, 2H), 4.48 (q, J = 7.1 Hz, 2H), 4.46 (s, 2H), 1.52 (s, 9H), 1.44 (t, J = 7.1 Hz, 3H), 1.42 (s, 9H).

12-(b): (6-{[4-(6-Ethoxpyridin-2-yl)benzyl](pyridin-2-ylsulfonyl)-aminomethyl}pyridin-2-ylamino)acetic acid

8.6 mL (112 mmol) of trifluoroacetic acid was added at room temperature to 8.6 mL solution of methylene chloride containing 590 mg (0.855 mmol) of the tert-butyl [tert-butoxycaibonyl(6-{[4-(6-ethoxypyridin-2-yl)benzyl] (pyridin-2-ylsulfonyl)aminomethyl}pyridin-2-yl)amino]acetate obtained in Example 12-(a) followed by stirring for 6 hours at room temperature. After completion of the reaction, the reaction solution was concentrated under reduced pressure followed by addition of water, adjusting the pH to 4.5 with 2 mol/L aqueous sodium hydroxide solution and dilute hydrochloric acid, and extraction with ethyl acetate. The organic layer was washed with saturated aqueous sodium chloride solution and dried with anhydrous magnesium sulfate followed by concentration under reduced pressure to obtain 357 mg of the title compound in the form of a white foam (yield: 78%).
mass spectrum (FAB, m/z): 534 ($M^+$+1).
$^1$H-NMR spectrum (DMSO-$d_6$, δ ppm): 12.59 (brs, 0.5H), 8.67 (d, J = 4.7 Hz, 1H), 8.01-7.95 (m, 3H), 7.85 (d, J = 7.7 Hz, 1H), 7.76 (dd, J = 8.4, 7.5 Hz, 1H), 7.61 (dd, J = 7.2,4.7 Hz, 1H), 7.50 (d, J = 7.5 Hz, 1H), 7.36-7.27 (m, 3H), 6.75 (d, J = 8.4 Hz, 1H), 6.44 (s, 1H), 6.37 (s, 1H), 4.72 (s, 2H), 4.42 (q, J = 7.1 Hz, 2H), 4.33 (s, 2H), 3.87 (s, 2H), 1.37 (t, J = 7.1 Hz, 3H).

[Example 13]

Ethyl (6-{[3'-1(-propynyl)biphenyl-4-ylmethyl](thiophen-2-ylsulfonyl)-aminomethyl}pyridin-2-ylamino)acetate

333 mg (1.50 mmol) of 3'-(1-propynyl)biphenyl-4-yl methanol obtained according to the same method as Reference Example 13, 740 μL (3.00 mmol) of tri-n-butylphosphine and 517 mg (3.00 mmol) of N,N,N',N'-tetramethylazodicarboxamide were added to 8.0 mL solution of tetrahydrofuran containing 533 mg (1.50 mol) of the ethyl {6-[(tbiophen-2-ylsulfbnyl)aminomethyl]pyridin-2-ylamino} acetate obtained in Reference Example 9-(b) followed by stirring for 7 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate =1:0→1:1 (V/V)) and the fractions containing the target product were concentrated under reduced pressure to obtain 806 mg of the title compound in the form of a colorless oil (yield: 96%).
mass spectrum (CI, m/z): 560 ($M^+$+1).
$^1$H-NMR spectrum ($CDCl_3$, δ ppm): 7.60-7.59 (m, 1H), 7.51 (dd, J = 5.0, 1.3 Hz, 1H), 7.48-7.43 (m, 4H), 7.36-7.27 (m,

5H), 7.01 (dd, J = 5.0, 3.8 Hz, 1H), 6.54 (d, J = 6.9 Hz, 1H), 6.29 (d, J = 8.0 Hz, 1H), 4.78 (t, J = 5.4 Hz, 1H), 4.60 (s, 2H), 4.33 (s, 2H), 4.21 (q, J = 7.1 Hz, 2H), 3.99 (d, J = 5.4 Hz, 2H), 2.07 (s, 3H), 1.27 (t, J = 7.1 Hz, 3H).

[Example 14]

(6-{[3'-(1-Propynyl)biphenyl-4-ylmethyl](thiophen-2-ylsulfonyl)-aminomethyl}pyridin-2-ylamino)acetic acid

6.0 mL (6.0 mmol) of 1 mol/L aqueous sodium hydroxide solution was added to 6.0 mL solution of ethanol containing 800 mg (1.43 mmol) of the ethyl (6-{[3'-(1-propynyl)biphenyl-4-ylmethyl](thiophen-2-ylsulfonyl)-aminomethyl} pyridin-2-ylamino)acetate obtained in Example 13 followed by stirring for 4 hours at room temperature. After completion of the reaction, water was added to the reaction solution and the pH was adjusted to 4.5 with 1 mol/L hydrochloric acid followed by extracting with ethyl acetate. The organic layer was washed with saturated aqueous sodium chloride solution and dried with anhydrous sodium sulfate followed by concentration under reduced pressure. The concentrate was dissolved in 10 mL of ethyl acetate and 10 mL of n-hexane was added at 50°C followed by stirring until the temperature of the solution reached room temperature over the course of 1.5 hours. The precipitated solid was collected by filtration and dried under reduced pressure to obtain 620 mg of the title compound in the form of a white solid (yield: 82%).
mass spectrum ($ESI^+$, m/z): 532 ($M^+$+1).
$^1$H-NMR spectrum (DMSO-$d_6$, δ ppm): 12.39 (brs, 0.9H), 7.91 (dd, J = 5.0, 1.3 Hz, 1H), 7.64-7.59 (m, 4H), 7.54 (dd, J = 3.8, 1.3 Hz, 1H), 7.43 (dd, J = 7.7, 7.7 Hz, 1H), 7.38-7.32 (m, 3H), 7.26 (dd, J = 8.3, 7.2 Hz, 1H), 7.13 (dd, J = 5.0, 3.8 Hz, 1H), 6.80 (t, J = 5.8 Hz, 1H), 6.41 (d, J = 8.3 Hz, 1H), 6.35 (d, J = 7.2 Hz, 1H), 4.59 (s, 2H), 4.18 (s, 2H), 3.84 (d, J = 5.8 Hz, 2H), 2.07 (s, 3H).

[Example 15]

Ethyl (6-{(benzenesulfonyl)[3'-(1-propynyl)biphenyl-4-ylmethyl)-aminnmethyl}pyridin-2-ylamino)acetate

333 mg (1.50 mmol) of 3'-(1-propynyl)biphenyl-4-yl methanol obtained according to the same method as Reference Example 13, 740 μL (3.00 mmol) of tri-n-butylphosphine and 517 mg (3.00 mmol) of N,N,N',N'-tetramethylazodicarboxamide were added to 8.0 mL solution of tetrahydrofuran containing 524 mg (1.50 mol) of the ethyl {6-[(benzenesulfonyl)aminomethyl]pyridin-2-ylamino}acetate obtained in Reference Example 10-(b) followed by stirring for 2 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 3:1→1:1 (V/V)) and the fractions containing the target product were concentrated under reduced pressure to obtain 809 mg of the title compound in the form of a colorless oil (yield: 97%).
mass spectrum (CI, m/z): 554 ($M^+$+1).
$^1$H-NMR spectrum ($CDCl_3$, δ ppm): 7.78-7.75 (m, 2H), 7.59-7.58 (m, 1H), 7.53-7.40 (m, 6H), 7.37-7.25 (m, 5H), 6.48 (d, J = 7.0 Hz, 1H), 6.27 (d, J = 8.0 Hz, 1H), 4.74 (t, J = 5.2 Hz, 1H), 4.58 (s, 2H), 4.32 (s, 2H), 4.21 (q, J = 7.2 Hz, 2H), 3.90 (d, J = 5.2 Hz, 2H), 2.07 (s, 3H), 1.27 (t, J = 7.2 Hz, 3H).

[Example 16]

(6-{(Benzenesulfonyl)[3'-1-propynyl)biphenyl-4-ylmethyl]aminomethyl}-pyridin-2-ylamino)acetic acid

6.0 mL (6.0 mmol) of 1 mol/L aqueous sodium hydroxide solution was added to 6.0 mL solution of ethanol containing 804 mg (1.45 mmol) of the ethyl (6-{(benzenesulfonyl)[3'-(1-propynyl)biphenyl-4-ylmethyl]-aminomethyl}pyridin-2-ylamino)acetate obtained in Example 15 followed by stirring for 4 hours at room temperature. After completion of the reaction, water was added to the reaction solution and the pH was adjusted to 4.5 with 1 mol/L hydrochloric acid followed by extracting with ethyl acetate. The organic layer was washed with saturated aqueous sodium chloride solution and dried with anhydrous sodium sulfate followed by concentration under reduced pressure. The concentrate was dissolved in 10 mL of ethyl acetate, and 10 mL of n-hexane was added at 50°C followed by stirring until the temperature of the solution reached room temperature over the course of 2 hours. The precipitated solid was collected by filtration and dried under reduced pressure to obtain 724 mg of the title compound in the form of a white solid (yield: 95%).
mass spectrum ($ESI^+$, m/z): 526 ($M^+$+1).
$^1$H-NMR spectrum (DMSO-$d_6$, δ ppm): 12.40 (brs, 0.6H), 7.75-7.72 (m, 2H), 7.63-7.58 (m, 5H), 7.53-7.48 (m, 2H), 7.43 (dd, J = 7.7, 7.7 Hz, 1H), 7.37 (ddd, J = 7.7, I.4, 1.4 Hz, 1H), 7.33-7.30 (m, 2H), 7.23 (dd, J = 8.3, 7.2 Hz, 1H), 6.75 (t, J = 5.6 Hz, 1H), 6.37 (d, J = 8.3 Hz, 1H), 6.29 (d, J = 7.2 Hz, 1H), 4.59 (s, 2H), 4.17 (s, 2H), 3.77 (d, J = 5.6 Hz, 2H),

2.07 (s, 3H).

[Example 17]

Ethyl (6-{[3'-(1-propynyl)biphenyl-4-ylmethyl](thiophen-3-ylsulfonyl)-aminomethyl}pyridin-2-ylamino)acetate

178 mg (0.800 mmol) of 3'-{1-propynyl)biphenyl-4-yl methanol obtained according to the same method as Reference Example 13, 395 μL (1.60 mmol) of tri-n-butylphosphine and 276 mg (1.60 mmol) of N,N,N',N'-tetramethylazodicarboxamide were added to 4.0 mL solution of tetrahydrofuran containing 284 mg (0.800 mol) of the ethyl {6-[(thiophen-3-ylsulfonyl)aminomethyl]pyridin-2-ylaminol acetate obtained in Reference Example 11-(b) followed by stirring for 3 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 4:1→1:1 (V/V)) and the fractions containing the target product were concentrated under reduced pressure to obtain 432 mg of the title compound in the form of a colorless syrup (yield: 97%).
mass spectrum (CI, m/z): 560 ($M^+$+1).
$^1$H-NMR spectrum ($CDCl_3$, δ ppm): 7.80 (dd, J = 3.1,1.3 Hz, 1H), 7.60-7.59 (m, 1H), 7.50-7.45 (m, 3H), 7.36-7.28 (m, 6H), 7.17 (dd, J = 5.1, 1.3 Hz, 1H), 6.52 (d, J = 7.2 Hz, 1H), 6.31 (d, J = 8.0 Hz, 1H), 4.80 (t, J = 5.4 Hz, 1H), 4.61 (s, 2H), 4.32 (s, 2H), 4.21 (q, J = 7.2 Hz, 2H), 3.99 (d, J = 5.4 Hz, 2H), 2.08 (s, 3H), 1.27 (t, J = 7.2 Hz, 3H).

[Example 18]

(6-{[3'-(1-Propynyl)biphenyl-4-ylmethyl](thiophen-3-ylsulfonyl)-aminomethyl}pyridin-2-ylamino)acetic acid

3.5 mL (3.5 mmol) of 1 mol/L aqueous sodium hydroxide solution was added to 3.5 mL solution of ethanol containing 426 mg (0.762 mmol) of the ethyl (6-{[3'-(1-propynyl)biphenyl-4-ylmethyl](thiophen-3-ylsulfonyl)-aminomethyl} pyridin-2-ylamino)acetate obtained in Example 17 followed by stirring for 16 hours at room temperature. After completion of the reaction, water was added to the reaction solution and the pH was adjusted to 4.4 with 1 mol/L hydrochloric acid followed by extracting with ethyl acetate. The organic layer was washed with saturated aqueous sodium chloride solution and dried with anhydrous sodium sulfate followed by concentration under reduced pressure. 5 mL of ethyl acetate and 5 mL of n-hexane were added to the concentrate followed by heating to 50°C and stirring until the temperature of the solution reached room temperature over the course of 2 hours. The precipitated solid was collected by filtration and dried under reduced pressure to obtain 390 mg of the title compound in the form of a white solid (yield: 96%).
mass spectrum (CI, m/z): 532 ($M^+$+1).
$^1$H-NMR spectrum (DMSO-$d_6$, δ ppm): 12.46 (brs, 0.6H), 8.14 (dd, J = 3.0, 1.4 Hz, 1H), 7.66 (dd, J = 5.1, 3.0 Hz, 1H), 7.64-7.59 (m, 4H), 7.45-7.24 (m, 6H), 6.81 (t, J = 5.5 Hz, 1H), 6.40 (d, J = 8.2 Hz, 1H), 6.33 (d, J = 7.0 Hz, 1H), 4.58 (s, 2H), 4.16 (s, 2H), 3.84 (d, J = 5.5 Hz, 2H), 2.07 (s, 3H).

[Example 19]

(6-{(3-Fluorobenzenesulfonyl)[3'-(1-propynl)biphenyl-4-ylmethyl]-aminomethyl}pyridin-2-ylamino)acetic acid

19-(a): tert-Butyl [tert-butoxycarbonyl(6- {(3-fluorobenzenesulfonyl) [3'-(1-propynyl)biphenyl-4-ylmethyl]aminomethyl} pyridin-2-yl)amino]acetate

280 μL (2.01 mmol) of triethylamine and 150 μL (1.13 mmol) of 3-fluorobenzenesulfonyl chloride were added to 3.5 mL solution of methylene chloride containing 542 mg (1.00 mmol) of tert-butyl [tert-butoxycarbonyl-(6-{[3'-(1-propynyl)biphenyl-4-ylmethyl]aminomethyl}pyridin-2-yl)amino]acetate obtained in Reference Example 12(c) while cooling with ice followed by stirring for 2 hours at room temperature. After completion of the reaction, water was added to the reaction solution followed by extracting with methylene chloride. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 9:1→7:3 (V/V)) and the fractions containing the target product were concentrated under reduced pressure to obtain 673 mg of the title compound in the form of a white foam (yield: 96%).
mass spectrum (CI, m/z): 700 ($M^+$+1).
$^1$H-NMR spectrum ($CDCl_3$, δ ppm): 7.70 (d, J = 8.1 Hz, 1H), 7.59-7.58 (m, 1H), 7.53-7.32 (m, 9H), 7.27-7.19 (m, 3H), 6.87 (d, J = 7.3 Hz, 1H), 4.57 (s, 2H), 4.39 (s, 2H), 4.37 (s, 2H), 2.08 (s, 3H), 1.52 (s, 9H), 1.42 (s, 9H).

19-(b): (6-{(3-Fluorobenzenesulfonyl)[3'-(1-pronynyl)biphenyl-4-ylmethyl]-aminomethyl}pyridin-2-ylamino)acetic acid

5.0 mL (20 mmol) of 4 mol/L hydrochloric acid was added to 5.0 mL solution of tetrahydrofuran containing 595 mg (0.850 mmol) of the tert-butyl [tert-butoxycarbonyl(6-{(3-fluorobenzensulfonyl)[3'-(1-propynyl)biphenyl-4-ylmethyl] -aminomethyl}pyridin-2-yl)amino]acetate obtained in Example 19-(a) followed by stirring for 5 hours at 70°C. After completion of the reaction, the pH was adjusted to 4.5 with 1 mol/L aqueous sodium hydroxide solution followed by extracting with ethyl acetate. The organic layer was washed with saturated aqueous sodium chloride solution and dried with anhydrous sodium sulfate followed by concentration under reduced pressure. 10 mL of ethyl acetate and 5 mL of n-hexane were added to the concentrate followed by heating to 50°C and stirring until the temperature of the solution reached room temperature over the course of 2 hours. The precipitated solid was collected by filtration and dried under reduced pressure to obtain 429 mg of the title compound in the form of a white solid (yield: 93%).
mass spectrum ($ESI^+$, m/z): 544 ($M^+$+1).
$^1$H-NMR spectrum (DMSO-$d_6$, $\delta$ ppm): 12.41 (brs, 0.9H), 7.65-7.60 (m, 4H), 7.58-7.50 (m, 2H), 7.46-7.34 (m, 6H), 7.25 (dd, J = 8.3, 7.2 Hz, 1H), 6.79 (t, J = 5.7 Hz, 1H), 6.38 (d, J = 8.3 Hz, 1H), 6.32 (d, J = 7.2 Hz, 1H), 4.67 (s, 2H), 4.19 (s, 2H), 3.74 (d, J = 5.7 Hz, 2H), 2.07 (s, 3H).

[Example 20]

Isopropyl (6-{[3'-(1-propynyl)biphenyl-4-ylmethyl](pyridin-2-ylsulfonyl)-aminomethyl}pyridin-2-ylamino)acetate

640 mg (2.88 mmol) of 3'-(1-propynyl)biphenyl-4-yl methanol obtained according to the same method as Reference Example 13, 1.42 mL (5.76 mmol) of tri-n-butylphosphine and 992 mg (5.76 mmol) of N,N,N',N'-tetramethylazodicarboxamide were added to 15.0 mL solution of tetrahydrofuran containing 1.05 g (2.88 mol) of the isopropyl (6-[(pyridin-2-ylsulfonyl)aminomethyl]pyridin-2-ylamino) acetate obtained in Reference Example 14 followed by stirring for 3 hours at room temperature. After completion of the reaction, water was added to the reaction solution followed by extracting with ethyl acetate. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 3:2$\rightarrow$2:3 (V/V)) and the fractions containing the target product were concentrated under reduced pressure to obtain 1.59 g of the title compound in the form of a colorless syrup (yield: 97%).
mass spectrum (CI, m/z): 569 ($M^+$+1).
$^1$H-NMR spectrum ($CDCl_3$, $\delta$ ppm): 8.62 (ddd, J = 4.7, 1.7, 1.0 Hz, 1H), 7.83 (ddd, J = 7.7, 1.0, 1.0 Hz, 1H), 7.76 (ddd, J = 7.7, 7.7, 1.7 Hz, 1H), 7.60-7.58 (m, 1H), 7.47-7.43 (m, 3H), 7.38 (ddd, J = 7.7, 4.7, 1.0 Hz, 1H), 7.36-7.32 (m, 4H), 7.23 (dd, J = 8.2, 7.3 Hz, 1H), 6.50 (d, J = 7.3 Hz, 1H), 6.22 (d, J = 8.2 Hz, 1H), 5.09 (sep, J = 6.3 Hz, 1H), 4.79 (s, 2H), 4.70 (t, J = 5.3 Hz, 1H), 4.42 (s, 2H), 3.92 (d, J = 5.3 Hz, 2H), 2.08 (s, 3H), 1.26 (d, J = 6.3 Hz, 6H).

The compounds used in the examples were synthesized in the manner described below.

[Reference Example 1]

Ethyl {6-[(pyridin-2-ylsulfonyl)aminomethyl]pyridin-2-ylamino}acetate 1-(a): tert-Butyl [tert-butoxycarbonyl(6-ethoxycarbonylpyridin-2-yl)amino]acetate

300 mL solution of N,N-dimethylformamide containing 81.2 g (0.305 mol) of ethyl 6-tert-butoxycarbonylaminopyridine-2-carboxylate (see WO2006/074884) were dropped into 362 mL solution of N,N-dimethylformamide containing 15.7 g (0.360 mol) of sodium hydride (55% by weight mineral oil dispersion) over the course of 20 minutes while cooling with ice in an argon atmosphere followed by stirring for 1 hour at room temperature. Next, 54.0 mL (0.366 mol) of tert-butyl bromoacetate were dropped in over the course of 10 minutes while cooling with ice followed by additionally stirring for 1 hour at room temperature. After completion of the reaction, an aqueous solution obtained by dissolving 1.77 g (33.0 mmol) of ammonium chloride in 300 mL of water was added to the reaction solution followed by extracting with toluene. The organic layer was washed with saturated aqueous sodium chloride solution and dried with anhydrous magnesium sulfate followed by concentration under reduced pressure. The residue was subjected to silica gel column chromatography (elution solvent: n-hexane:ethyl acetate = 9:1$\rightarrow$4:1 (V/V)) and the fractions containing the target product were concentrated under reduced pressure to obtain 108 g of the title compound in the form of a pale yellow oil (yield: 93%).
mass spectrum (CI, m/z): 381 ($M^+$+1).
$^1$H-NMR spectrum ($CDCl_3$, $\delta$ ppm): 8.04 (d, J = 7.8 Hz, 1H), 7.81 (dd, J = 7.6, 1.5 Hz, 1H), 7.76 (dd, J = 7.8, 7.6 Hz, 1H), 4.67 (s, 2H), 4.40 (q, J = 7.1 Hz, 2H), 1.52 (s, 9H), 1.45 (s, 9H), 1.40 (t, J = 7.1 Hz, 3H).

1-(b): tert-Butyl [tert-butoxycarbonyl(6-hydroxymethylpyridin-2-yl)amino]acetate

195 mL solution of ethanol containing 34.6 g (0.312 mol) of calcium chloride were dropped into 195 mL solution of ethanol containing 98.8 g (0.260 mol) of the tert-butyl [tert-butoxycarbonyl(6-ethoxycarbonylpyridin-2-yl)amino]acetate obtained in Reference Example 1-(a) over the course of 20 minutes while cooling with ice. After completion of dropping, 105 mL (0.315 mol) of 3 mol/L sodium borohydride/tetraethylene glycol dimethyl ether solution were dropped in over the course of 20 minutes at 35°C or lower followed by additionally stirring for 15 minutes at room temperature. After completion of the reaction, the reaction solution was dropped into a mixed solution of 17.8 mL of acetic acid and 195 mL of water over the course of 10 minutes while cooling with ice followed by stirring for 1 hour at room temperature. Next, 315 mL of water was added followed by extracting with toluene. The organic layer was sequentially washed with saturated aqueous sodium bicarbonate solution, water and saturated aqueous sodium chloride solution and then concentrated under reduced pressure. The residue was subjected to silica gel column chromatography (elution solvent: n-hexane:ethyl acetate = 4:1→3:2 (V/V)) and the fractions containing the target product were concentrated under reduced pressure to obtain 81.1 g of the title compound in the form of a pale yellow oil (yield: 92%).
mass spectrum (CI, m/z): 339 ($M^+$+1).
$^1$H-NMR spectrum ($CDCl_3$, $\delta$ ppm): 7.74 (d, J = 8.2 Hz, 1H), 7.63 (dd, J = 8.2, 7.4 Hz, 1H), 6.93-6.98 (m, 1H), 4.68-4.65 (m, 2H), 4.54 (s, 2H), 3.39 (t, J = 5.3 Hz, 1H), 1.54 (s, 9H), 1.46 (s, 9H).

1-(c): tert-Butyl [tert-butoxycarbonyl(6-formylpyridin-2-ylamino]acetate

50 mL solution of methylene chloride containing 10.0 g (29.6 mmol) of the tert-butyl [tert-butoxycarbonyl(6-hydroxymethylpyridin-2-yl)amino] acetate obtained in Reference Example 1-(b) were dropped into 130 mL solution of methylene chloride containing 12.9 g (30.4 mmol) of Dess-Martin reagent over the course of 20 minutes while cooling with ice in an argon atmosphere. Following completion of dropping, the reaction solution was stirred for 2 hours at room temperature. After completion of the reaction, 305 mL of 0.1% by weight sodium thiosulfate solution was added to the reaction solution followed by extracting with methylene chloride. The organic layer was sequentially washed with 0.5 mol/L aqueous sodium hydroxide solution and saturated aqueous sodium chloride solution, dried with anhydrous magnesium sulfate, and then concentrated under reduced pressure to obtain 9.61 g of the title compound in the form of a pale yellow oil nearly quantitatively.
mass spectrum (EI, m/z): 336 ($M^+$).
$^1$H-NMR spectrum (DMSO-$d_6$, $\delta$ ppm): 9.82 (s, 1H), 8.11-7.99 (m, 2H), 7.68 (dd, J = 6.6, 1.5 Hz, 1H), 4.58 (s, 2H), 1.48 (s, 9H), 1.42 (s, 9H).

1-(d): tert-Butyl

[tert-butoxycarbonyl(6-hydroxyiminomethylpyridin-2-yl)amino]acetate

0.650 g (9.35 mmol) and 3.5 mL (43 mmol) of pyridine were added to 29 mL solution of methanol containing 2.88 g (8.56 mmol) of the tert-butyl [tert-butoxycarbonyl(6-formylpyridin-2-yl)amino]acetate obtained in Reference Example 1-(c) followed by stirring for 1 hour at room temperature. After completion of the reaction, the reaction solution was concentrated under reduced pressure. Ethyl acetate was added to the resulting residue followed by sequentially washing with 5% by weight aqueous potassium hydrogen sulfate, saturated aqueous sodium bicarbonate solution and saturated aqueous sodium chloride solution, drying with anhydrous magnesium sulfate, and then concentration under reduced pressure. The residue was subjected to silica gel column chromatography (elution solvent: n-hexane:ethyl acetate = 3:2 (V/V)) and the fractions containing the target product were concentrated under reduced pressure to obtain 2.76 g of the title compound in the form of a colorless oil (yield: 92%).
mass spectrum (EI, m/z): 351 ($M^+$).
$^1$H-NMR spectrum ($CDCl_3$, $\delta$ ppm): 8.06 (s, 1H), 7.91 (s, 1H), 7.85 (d, J = 8.2 Hz, 1H), 7.65 (dd, J = 8.2, 7.6 Hz, 1H), 7.47 (dd, J = 7.6, 0.7 Hz, 1H), 4.59 (s, 2H), 1.53 (s, 9H), 1.45 (s, 9H).

1-(e): tert-Butyl

[(6-aminomethylpyridin-2-yl)tert-butoxycarbonylamino]acetate

0.98 g of 10% by weight palladium-activated charcoal (water content: 50% by weight) was added to 49 mL solution of ethanol containing 2.75 g (7.83 mmol) of the tert-butyl [tert-butoxycarbonyl(6-hydroxyiminomethylpyridin-2-yl)amino] acetate obtained in Reference Example 1-(d) followed by stirring for 1 hour at room temperature in a hydrogen atmosphere at 1 atm. After completion of the reaction, insoluble substances were collected by filtration and the filtrate

was concentrated under reduced pressure to obtain 2.48 g of the title compound in the form of a colorless oil (yield: 94%).
mass spectrum (CI, m/z): 338 ($M^+$+1).
$^1$H-NMR spectrum ($CDCl_3$, δ ppm): 7.68 (d, J = 8.3 Hz, 1H), 7.58 (dd, J = 8.3, 7.4 Hz, 1H), 6.91 (d, J = 7.4 Hz, 1H), 4.57 (s, 2H), 3.85 (s, 2H), 1.53 (s, 9H), 1.46 (s, 9H).

1-(f): tert-Butyl (tert-butoxycarbonyl{6-[(pyridin-2-ylsulfonyl)aminomethyl]pyridin-2-yl}amino)acetate

12 mL solution of methylene chloride containing 1.20 g (3.56 mmol) of the tert-butyl [(6-aminomethylpyridin-2-yl)tert-butoxycarbonylamino]acetate obtained in Reference Example 1-(e) and 2.24 mL (16.2 mmol) of triethylamine were added to 14 mL solution of methylene chloride containing 0.640 g (3.60 mmol) of 2-pyridylsulfonyl chloride followed by stirring for 0.5 hours at room temperature. After completion of the reaction, a 5% by weight aqueous potassium hydrogen sulfate solution was added to the reaction solution followed by extracting with methylene chloride. The organic layer was sequentially washed with saturated aqueous sodium bicarbonate solution and saturated aqueous sodium chloride solution, dried with anhydrous magnesium sulfate, and then concentrated under reduced pressure. The residue was subjected to silica gel column chromatography (elution solvent: n-hexane:ethyl acetate =1:1 (V/V)) and the fractions containing the target product were concentrated under reduced pressure to obtain 1.46 g of the title compound in the form of a white solid (yield: 86%).
mass spectrum (APCI, m/z): 479 ($M^+$+1).
$^1$H-NMR spectrum ($CDCl_3$, δ ppm): 8.56 (ddd, J = 4.7, 1.7, 0.9 Hz, 1H), 7.97 (ddd, J = 7.8, 1.1, 0.9 Hz, 1H), 7.84 (ddd, J = 7.8, 7.7, 1.7 Hz, 1H), 7.68 (d, J = 8.4 Hz, 1H), 7.52 (dd, J = 8.4, 7.4 Hz, 1H), 7.40 (ddd, J = 7.7, 4.7, 1.1 Hz, 1H), 6.84 (dd, J = 7.4, 0.5 Hz, 1H), 5.86 (t, J = 5.6 Hz, 1H), 4.48 (s, 2H), 4.36 (d, J = 5.6 Hz, 2H), 1.53 (s, 9H), 1.45 (s, 9H).

1-(g): Ethyl {6-[(pyridin-2-ylsulfonyl)aminomethyl]pyridin-2-ylamino}acetate

37.5 mL (75.0 mmol) of 2 mol/L hydrogen chloride/ethanol solution was added to 3.59 g (7.50 mmol) of tert-butyl (tert-butoxycarbonyl {6-[(pyridin-2-ylsulfonyl)aminomethyl]pyridin-2-yl}amino)acetate obtained according to the same method as Reference Example 1-(f) followed by stirring for 3 hours while heating to reflux. After completion of the reaction, water was added to the reaction solution followed by neutralizing with 1 mol/L aqueous sodium hydroxide solution and extracting with ethyl acetate. The organic layer was washed with saturated aqueous sodium chloride solution, dried with anhydrous magnesium sulfate and then concentrated under reduced pressure to obtain 2.17 g of the title compound in the form of a brown oil (yield: 83%).
mass spectrum (CI, m/z): 351 ($M^+$+1).
$^1$H-NMR spectrum (DMSO-$d_6$, δ ppm): 8.71 (ddd, J = 4.8, 1.8, 0.8 Hz, 1H), 8.18 (brs, 0.1H), 8.05 (ddd, J = 7.8, 7.6, 1.8 Hz, 1H), 7.91 (ddd, J = 7.8, 1.0, 0.8 Hz, 1H), 7.64 (ddd, J = 7.6, 4.6, 1.0 Hz, 1H), 7.33 (dd, J = 8.1, 7.2 Hz, 1H), 6.86 (t, J = 6.1 Hz, 0.2H), 6.52 (d, J = 7.2 Hz, 1H), 6.39 (d, J = 8.1 Hz, 1H), 4.08 (q, J = 7.1 Hz, 2H), 4.01 (s, 2H), 3.95 (s, 2H), 1.16 (t, J = 7.1 Hz, 3H).

[Reference Example 2]

Ethyl {6-[(pyridin-3-ylsulfonyl)aminomethyl]pyridin-2-ylamino}acetate 2-(a): tert-Butyl (tert-butoxycarbonyl{6-[(pyridin-3-ylsulfonyl)aminomethyl]pyridin-2-yl}amino)acetate

1.45 g of the title compound were obtained in the form of a colorless oil by performing the reaction and post-treatment in accordance with Reference Example 1-(f) with the exception of using 1.20 g (3.56 mmol) of tert-butyl [(6-aminomethylpyridin-2-yl)tert-butoxycarbonylamino]acetate obtained according to the same method as Reference Example 1-(e) and using 640 mg (3.60 mmol) of 3-pyridylsulfonyl chloride in place of 2-pyridylsulfonyl chloride (yield: 85%).
mass spectrum (CI, m/z): 479 ($M^+$+1).
$^1$H-NMR spectrum ($CDCl_3$, δ ppm): 9.06 (d, J = 2.2 Hz, 1H), 8.71 (dd, J = 4.6, 1.5 Hz, 1H), 8.13-8.08 (m, 1H), 7.68 (d, J = 8.2 Hz, 1H), 7.52 (dd, J = 8.2, 7.4 Hz, 1H), 7.38-7.32 (m, 1H), 6.77 (d, J = 7.4 Hz, 1H), 5.80 (t, J = 5.1 Hz, 1H), 4.40 (s, 2H), 4.24 (d, J = 5.1 Hz, 2H), 1.53 (s, 9H), 1.46 (s, 9H).

2-(b): Ethyl {6-[(pyridin-3-ylsulfonyl)aminomethyl)pyridin-2-ylamino}acetate

686 mg of the title compound were obtained in the form of a brown oil by performing the reaction and post-treatment in accordance with Reference Example 1-(g) with the exception of using 1.00 g (2.09 mmol) of tert-butyl (tert-butoxycarbonyl{6-[(pyridin-3-ylsulfonyl)aminomethyl]pyridin-2-yl}amino)acetate obtained according to the same method as Reference Example 2-(a) in place of tert-butyl (tert-butoxycarbonyl{6-[(pyridin-2-ylsulfonyl)aminomethyl]pyridin-2-yl}amino)acetate, and using 10.4 mL (20.8 mmol) of 2 mol/L hydrogen chloride/ethanol solution (yield: 94%).

$^1$H-NMR spectrum ($CDCl_3$, δ ppm): 9.06 (dd, J = 2.3,0.7 Hz, 1H), 8.71 (dd, J = 4.9, 1.6 Hz, 1H), 8.09 (ddd, J = 8.0, 2.3, 1.6 Hz, 1H), 7.35 (ddd, J = 8.0, 4.9, 0.7 Hz, 1H), 7.28 (dd, J = 8.3, 7.3 Hz, 1H), 6.38 (d, J = 7.3 Hz, 1H), 6.29 (d, J = 8.3 Hz, 1H), 5.95 (t, J = 5.4 Hz, 1H), 4.96 (t, J = 5.4 Hz, 1H), 4.27 (q, J = 7.2 Hz, 2H), 4.14 (d, J = 5.4 Hz, 2H), 4.03 (d, J = 5.4 Hz, 2H), 1.32 (t, J = 7.2 Hz, 3H).

[Reference Example 3]

3'-(1-Propenyl)biphenyl-4-ylmethanol

3-(a): 3'-(1-Propenyl)biphenyl-4-ylcarbaldehyde

27.5 mL of toluene and 1.65 mL of water were added to 500 mg (1.91 mmol) of 3-bromobiphenyl-4-ylcarbaldehyde (see Journal of Organic Chemistry, 68, 247 (2003)) followed by the addition of 1.63 g (7.68 mmol) of tripotassium phosphate and 656 mg (7.64 mmol) of 1-propenylboronic acid and placing the mixture in a nitrogen gas atmosphere. Moreover, 6.2 mg (0.028 mmol) of palladium acetate and 20.2 mg (0.0563 mmol) of butyl di-1-adamantylphosphine were added followed by stirring for 4.5 hours at 100°C in a nitrogen gas atmosphere. After completion of the reaction, water was added to the reaction solution followed by extracting with ethyl acetate. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 4:1 (V/V)) and the fractions containing the target product were concentrated under reduced pressure to obtain 420 mg of the title compound in the form of a slightly yellow oil (yield: 99%).
mass spectrum (CI, m/z): 223($M^+$+1).
$^1$H-NMR spectrum ($CDCl_3$, δ ppm): 10.06 (s, 1H), 7.98-7.92 (m, 2H), 7.79-7.72 (m, 2H), 7.59-7.55 (m, 1H), 7.49-7.42 (m, 1H), 7.41-7.37 (m, 2H), 6.48 (dd, J = 15.9, 1.5 Hz, 1H), 6.33 (dq, J = 15.9, 6.3 Hz, 1H), 1.92 (dd, J = 6.3, 1.5 Hz, 3H).

3-(b): 3' -Propenyl)biphenyl-4-ylmethanol

35.6 mg (0.941 mmol) of sodium borohydride was added to 4.6 mL solution of ethanol containing 417 mg (1.88 mmol) of the 3'-(1-propenyl)biphenyl-4-ylcarbaldehyde obtained in Reference Example 3-(a) at room temperature followed by stirring for 45 minutes at the same temperature. After completion of the reaction, saturated aqueous ammonium chloride solution was added to the reaction solution followed by extracting with ethyl acetate. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 7:3 (V/V)) and the fractions containing the target product were concentrated under reduced pressure to obtain 401 mg of the title compound in the form of a white solid (yield: 95%).
mass spectrum (EI, m/z): 224 ($M^+$).
$^1$H-NMR spectrum ($CDCl_3$, δ ppm): 7.63-7.56 (m, 2H), 7.55-7.52 (m, 1H), 7.47-7.29 (m, 5H), 6.47 (dd, J =15.9, 1.5 Hz, 1H), 6.31 (dq, J = 15.9, 6.6 Hz, 1H), 4.74 (d, J = 5.7 Hz, 2H), 1.91 (dd, J = 6.6, 1.5 Hz, 3H), 1.70 (t, J = 5.7 Hz, 1H).

[Reference Example 4]

3'-(1-Propynyl)biphenyl-4-ylmethanol

4-(a):3'-(1-Propynyl)biphenyl-4-ylcarbaldehyde

10 mL solution of toluene containing 1.04 g (3.98 mmol) of 3'-bromobiphenyl-4-ylcarbaldehyde were degassed under reduced pressure followed by substituting with argon gas. Next, 231 mg (0.200 mmol) of tetrakis(triphenylphosphine)palladium and 1.46 mL (4.80 mmol) of tributyl(1-propynyl)tin were added followed by stirring for 7 hours at 110°C in an argon gas atmosphere. After completion of the reaction, 60 mL of 0.8 mol/L aqueous potassium fluoride solution was added to the reaction solution followed by extracting with toluene. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 1:0→4:1 (V/V)) and the fractions containing the target product were concentrated under reduced pressure to obtain 660 mg of the title compound in the form of a pale yellow solid (yield: 75%).
mass spectrum (CI, m/z): 221 ($M^+$+1).
$^1$H-NMR spectrum ($CDCl_3$, δ ppm): 10.06 (s, 1H), 7.97-7.93 (m, 2H), 7.76-7.72 (m, 2H), 7.68-7.67 (m, 1H), 7.55-7.52 (m, 1H), 7.45-7.37 (m, 2H), 2.08 (s, 3H).

4-(b): 3'-(1-Propynyl)biphenyl-4-ylmethanol

588 mg of the title compound were obtained in the form of a slightly yellowish white solid by performing the reaction and post-treatment in accordance with Reference Example 3-(b) with the exception of using 723 mg (3.28 mmol) of 3'-(1-propynyl)biphenyl-4-ylcarbaldehyde obtained according to the same method as Reference Example 4-(a) in place of 3'-(1-propenyl)biphenyl-4-ylcarbaldehyde, and using 62.2 mg (1.64 mmol) of sodium borohydride (yield: 81%).
mass spectrum (EI, m/z): 222 ($M^+$).
$^1$H-NMR spectrum ($CDCl_3$, $\delta$ ppm): 7.63-7.62 (m, 1H), 7.60-7.56 (m, 2H), 7.51-7.47 (m, 1H), 7.46-7.42 (m, 2H), 7.38-7.32 (m, 2H), 4.75 (d, J = 6.0 Hz, 2H), 2.07 (s, 3H), 1.68 (t, J = 6.0 Hz, 1H).

[Reference Example 5]

3'-Ethoxybiphenyl-4-ylmethanol

15 mL of toluene, 15 mL of ethanol and 4.5 mL (9.0 mmol) of 2 mol/L aqueous sodium carbonate solution were added to 1.21 g (6.02 mmol) of 3-bromophenetole followed by degassing under reduced pressure and substituting with argon gas. Next, 1.37 g (9.02 mmol) of 4-(hydroxymethyl)phenylboronic acid and 347 mg (0.300 mmol) of tetrakis(triphenylphosphine)palladium were added followed by stirring for 4 hours at 100°C in an argon gas atmosphere. After completion of the reaction, the reaction solution was concentrated under reduced pressure and water was added to the residue followed by extracting with ethyl acetate. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 9:1→7:3 (V/V)) and the fractions containing the target product were concentrated under reduced pressure to obtain 1.23 g of the title compound in the form of a pale yellow oil (yield: 90%).
mass spectrum (CI, m/z): 229 ($M^+$+1).
$^1$H-NMR spectrum ($CDCl_3$, $\delta$ ppm): 7.61-7.56 (m, 2H), 7.46-7.41 (m, 2H), 7.34 (dd, J = 8.0, 8.0 Hz, 1H,), 7.18-7.11 (m, 2H), 6.91-6.87 (m, 1H), 4.74 (d, J = 5.9 Hz, 2H), 4.10 (q, J = 7.0 Hz, 2H), 1.67 (t, J = 5.9 Hz, 1H), 1.45 (t, J = 7.0 Hz, 3H).

[Reference Example 6]

Hexyl {6-[(pyridin-2-ylulfonylaminomethyl]pyridin-2-ylamino}acetate

0.56 mL (10 mmol) of concentrated sulfuric acid was added to 6.0 mL solution of n-hexanol containing 957 mg (2.00 mmol) of tert-butyl (tert-butoxycarbonyl {6-[(pyridin-2-ylsulfonyl)aminomethyl]pyridin-2-ylamino}acetate obtained according to the same method as Reference Example 1-(f) followed by stirring for 8 hours at 100°C. After completion of the reaction, the reaction solution was poured into a saturated aqueous sodium bicarbonate solution followed by extracting with ethyl acetate. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 1:1→3:7 (V/V)) and the fractions containing the target product were concentrated under reduced pressure to obtain 658 mg of the title compound in the form of a slightly yellow oil (yield: 81%).
$^1$H-NMR spectrum ($CDCl_3$, $\delta$ ppm): 8.62 (ddd, J = 4.6, 1.8, 1.0 Hz, 1H), 7.97 (ddd, J = 7.7, 1.2, 1.0 Hz, 1H), 7.84 (ddd, J = 7.7, 7.7, 1.8 Hz, 1H), 7.41 (ddd, J = 7.7, 4.6, 1.2 Hz, 1H), 7.29 (dd, J = 8.4, 7.4 Hz, 1H), 6.44 (d, J = 7.4 Hz, 1H), 6.28 (d, J = 8.4 Hz, 1H), 6.02 (t, J = 5.3 Hz, 1H), 4.92 (t, J = 5.3 Hz, 1H), 4.25 (d, J = 5.3 Hz, 2H), 4.18 (t, J = 6.7 Hz, 2H), 4.08 (d, J = 5.3 Hz, 2H), 1.71-1.61 (m, 2H), 1.39-1.26 (m, 6H), 0.91-0.87 (m, 3H).

[Reference Example 7]

tert-Butyl (tert-butoxycarbonyl{6-[(3'-ethoxybiphenyl-4-ylmethyl)-aminomethyl]pyridin-2-ylamino}acetate

7-(a): 3'-Ethoxybiphenyl-4-ylcarbaldehyde

4.08 g of the title compound were obtained in the form of a colorless oil by performing the reaction and post-treatment in accordance with Reference Example 5 with the exception of respectively using 4.20 g (22.7 mmol) of 4-bromobenzaldehyde in place of 3-bromophenetole and 3.13 g (18.9 mmol) of 3-ethoxyhenylboronic acid in place of 4-(hydroxymethyl)phenylboronic acid, and using 28.4 mL (56.8 mmol) of 2 mol/L aqueous sodium carbonate solution and 2.18 g (1.89 mmol) of tetrakis(triphenylphosphine)palladium (yield: 95%).
mass spectrum (CI, m/z): 227 ($M^+$+1).

$^1$H-NMR spectrum ($CDCl_3$, δ ppm): 10.06 (s, 1H), 7.97-7.93 (m, 2H), 7.76-7.73 (m, 2H), 7.38 (dd, J = 8.1, 7.9 Hz, 1H), 7.21 (ddd, J = 7.9, 2.0, 0.9 Hz, 1H), 7.16 (dd, J = 2.3, 2.0 Hz, 1H), 6.95 (ddd, J = 8.1, 2.3, 0.9 Hz, 1H), 4.11 (q, J = 6.9 Hz, 2H), 1.46 (t, J = 6.9 Hz, 3H).

7-(b):tert-Butyl(tert-butoxycarbonyl{6-[(3'-ethoxybiphenyl-4-ylmethyl)-aminomethyl]pyridin-2-yl}amino)acetate

2.46 g (10.9 mmol) of the 3'-ethoxybiphenyl-4-ylcarbaldehyde obtained in Reference Example 7-(a) was added to 12 mL solution of methylene chloride containing 4.02 g (11.9 mmol) of tert-butyl [(6-aminomethylpyridin-2-yl)tert-butoxycarbonylamino] acetate obtained according to the same method as Reference Example 1-(e) followed by stirring for 30 minutes at room temperature. Next, 3.25 g (15.3 mmol) of sodium triacetoxyborohydride was added while cooling with ice followed by stirring for 3.5 hours at the same temperature. After completion of the reaction, aqueous sodium bicarbonate solution was added followed by extracting with ethyl acetate. The organic layer was washed with saturated aqueous sodium chloride solution, dried with anhydrous potassium carbonate, and then concentrated under reduced pressure. The residue was subjected to silica gel column chromatography (elution solvent: n-hexane:ethyl acetate = 3:2→0:1 (V/V)) and the fractions containing the target product were concentrated under reduced pressure to obtain 3.68 g of the title compound in the form of a pale yellow oil (yield: 62%).
mass spectrum (CI, m/z): 548 ($M^+$+1).
$^1$H-NMR spectrum ($CDCl_3$, δ ppm): 7.69 (d, J = 8.2 Hz, 1H), 7.59 (dd, J = 8.2, 7.3 Hz, 1H), 7.57-7.53 (m, 2H), 7.43-7.39 (m, 2H), 7.33 (dd, J = 7.9,7.7 Hz, 1H), 7.16 (ddd, J = 7.7, 1.7, 0.9 Hz, 1H), 7.12 (dd, J = 2.3, 1.7 Hz, 1H), 6.97 (d, J = 7.3 Hz, 1H), 6.87 (ddd, J = 7.9, 2.3, 1.0 Hz, 1H), 4.57 (s, 2H), 4.10 (q, J = 7.1 Hz, 2H), 3.84 (s, 2H), 3.83 (s, 2H), 1.53 (s, 9H), 1.44 (t, J = 7.1 Hz, 3H), 1.42 (s, 9H).

[Reference Example 8]

4-(6- Ethoxypyridin-2-yl)phenylmethanol

284 mg of the title compound were obtained in the form of a white solid by performing the reaction and post-treatment in accordance with Reference Example 5 with the exception of using 0.49 g (2.4 mmol) of 2-bromo-6-ethoxypyridine (see US 2003/199440) in place of 3-bromophenetole, and using 0.59 g (3.9 mmol) of 4-(hydroxymethyl)phenylboronic acid, 1.7 mL (3.4 mmol) of 2 mol/L aqueous sodium carbonate solution, and 138 mg (0.119 mmol) of tetrakis(triphenylphosphine)palladium (yield: 51 %).
mass spectrum (CI, m/z): 230 ($M^+$+1).
$^1$H-NMR spectrum ($CDCl_3$, δ ppm): 8.05-8.01 (m, 2H), 7.62 (dd, J = 8.2, 7.4 Hz, 1H), 7.47-7.43 (m, 2H), 7.32 (dd, J = 7.4,0.6 Hz, 1H), 6.67 (dd, J = 8.2,0.6 Hz, 1H), 4.75 (d, J = 6.0 Hz, 2H), 4.49 (q, J = 7.1 Hz, 2H), 1.67 (t, J = 6.0 Hz, 1H), 1.44 (t, J = 7.1 Hz, 3H).

[Reference Example 9]

Ethyl {6-[(thiophen-2-ylsulfonyl)aminomethyl]pyridin-2-ylamino}acetate

9-(a): tert-Butyl (tert-butoxycarbonyl {6-[(thiophen-2-ylsulfonyl)aminomethyl]-pyridin-2-ylamino}acetate

1.61 g of the title compound were obtained in the form of a white solid by performing the reaction and post-treatment in accordance with Reference Example 1-(f) with the exception of using 1.35 g (4.00 mmol) of tert-butyl [(6-aminomethylpyridin-2-yl)tert-butoxycarbonylamino] acetate obtained according to the same method as Reference Example 1-(e), and using 731 mg (4.00 mmol) of 2-thiophenesulfonyl chloride in place of 2-pyridylsulfonyl chloride (yield: 84%).
mass spectrum (CI, m/z): 484 ($M^+$+1).
$^1$H-NMR spectrum ($CDCl_3$, δ ppm): 7.71 (d, J = 8.4 Hz, 1H), 7.57 (dd, J = 3.8,1.3 Hz, 1H), 7.56 (dd, J = 8.4, 7.4 Hz, 1H), 7.50 (dd, J = 5.0, 1.3 Hz, 1H), 7.01 (dd, J = 5.0,3.8 Hz, 1H), 6.83 (d, J = 7.4 Hz, 1H), 5.67 (t, J = 5.3 Hz, 1H), 4.45 (s, 2H), 4.27 (d, J = 5.3 Hz, 2H), 1.53 (s, 9H), 1.47 (s, 9H).

9-(b): Ethyl {6-[(thiophen-2-ylsulfonyl)aminomethyl]pyridin-2-ylamino} acetate

20 mL (40 mmol) of 2 mol/L hydrogen chloride/ethanol solution was added to 1.60 g (3.31 mmol) of the tert-butyl (tert-butoxycarbonyl {6-[(thiophen-2-ylsulfonyl)-aminomethyl]pyridin-2-ylamino} acetate obtained in Reference Example 9-(a) followed by stirring for 3 hours while heating to reflux. After completion of the reaction, the reaction solution was concentrated under reduced pressure and neutralized with saturated aqueous sodium bicarbonate solution followed

by extracting with ethyl acetate. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 7:3→1:1 (V/V)) and the fractions containing the target product were concentrated under reduced pressure to obtain 1.10 g of the title compound in the form of a colorless oil (yield: 93%).
mass spectrum (CI, m/z): 356 ($M^+$+1).
$^1$H-NMR spectrum ($CDCl_3$, δ ppm): 7.57 (dd, J = 3.8, 1.3 Hz, 1H), 7.51 (dd, J = 5.0, 1.3 Hz, 1H), 7.32 (dd, J = 8.3, 7.3 Hz, 1H), 7.01 (dd, J = 5.0, 3.8 Hz, 1H), 6.44 (dd, J = 7.3, 0.6 Hz, 1H), 6.32 (dd, J = 8.3, 0.6 Hz, 1H), 5.86 (t, J = 4.9 Hz, 1H), 4.96 (t, J = 5.3 Hz, 1H), 4.26 (q, J = 7.2 Hz, 2H), 4.18 (d, J = 4.9 Hz, 2H), 4.06 (d, J = 5.3 Hz, 2H), 1.32 (t, J = 7.2 Hz, 3H).

[Reference Example 10]

Ethyl {6-[(benzenesulfonyl)aminomethyl]pyridin-2-ylamino}acetate

10-(a): tert-Butyl ({6-[(benzenesulfonyl)aminomethyl]pyridine-2-yl}tert-butoxycarbonylamino)acetate

1.71 g of the title compound were obtained in the form of a slightly beige solid by performing the reaction and post-treatment in accordance with Reference Example 1-(f) with the exception of using 1.35 g (4.00 mmol) of tert-butyl [(6-aminomethylpyridin-2-yl)tert-butoxycarbonylamino] acetate obtained according to the same method as Reference Example 1-(e) and using 707 mg (4.00 mmol) of benzenesulfonyl chloride in place of 2-pyridylsulfonyl chloride (yield: 89%).
mass spectrum (CI, m/z): 478 ($M^+$+1).
$^1$H-NMR spectrum ($CDCl_3$, δ ppm): 7.86-7.83 (m, 2H), 7.67 (d, J = 8.4 Hz, 1H), 7.53-7.48 (m, 2H), 7.45-7.41 (m, 2H), 6.78 (dd, J = 7.4,0.6 Hz, 1H), 5.56 (t, J = 5.4 Hz, 1H), 4.41 (s, 2H), 4.19 (d, J = 5.4 Hz, 2H), 1.53 (s, 9H), 1.46 (s, 9H).

10-(b): Ethyl {6-[(benzenesulfonyl)aminomethyl]pyridin-2-ylamino}acetate

1.13 g of the title compound were obtained in the form of a white solid by performing the reaction and post-treatment in accordance with Reference Example 9-(b) with the exception of using 1.70 g (3.56 mmol) of the tert-butyl ({6-[(benzenesulfonyl)aminomethyl]pyridine-2-yl}tert-butoxycarbonylamino)acetate obtained in Reference Example 10-(a) in place of tert-butyl (tert-butoxycarbonyl{6-[(thiophen-2-ylsulfonyl)aminomethyl]pyridin-2-ylamino}acetat e, and 20 mL (40 mmol) of 2 mol/L hydrogen chloride/ethanol solution (yield: 91%).
mass spectrum (CI, m/z): 350 ($M^+$+1).
$^1$H-NMR spectrum ($CDCl_3$, δ ppm): 7.87-7.84 (m, 2H), 7.53-7.42 (m, 3H), 7.28 (dd, J = 8.3, 7.3 Hz, 1H), 6.39 (dd, J = 7.3, 0.6 Hz, 1H), 6.30 (dd, J = 8.3, 0.6 Hz, 1H), 5.73 (t, J = 4.9 Hz, 1H), 4.92 (t, J = 5.2 Hz, 1H), 4.26 (q, J = 7.2 Hz, 2H), 4.09 (d, J = 4.9 Hz, 2H), 4.04 (d, J = 5.2 Hz, 2H), 1.32 (t, J = 7.2 Hz, 3H).

[Reference Example 11]

Ethyl {6-[(thiophen-3-ylsulfonyl)aminomethyl]pyridin-2-ylamino}acetate

11-(a): tert-Butyl (tert-butoxycarbonyl{6-[(thiophen-3-ylsulfonyl)-aminomethyl]pyridine-2-yl}amino)acetate

1.64 g of the title compound were obtained in the form of a slightly yellowish white solid by performing the reaction and post-treatment in accordance with Reference Example 1-(f) with the exception of using 1.35 g (4.00 mmol) of tert-butyl [(6-aminomethylpyridin-2-yl)tert-butoxycarbonylamino]acetate obtained according to the same method as Reference Example 1-(e) and using 731 mg (4.00 mmol) of 3-thiophenesulfonyl chloride in place of 2-pyridylsulfonyl chloride (yield: 85%).
mass spectrum (CI, m/z): 484 ($M^+$+1).
$^1$H-NMR spectrum ($CDCl_3$, δ ppm): 7.93 (dd, J = 2.9,1.4 Hz, 1H), 7.69 (d, J = 8.3 Hz, 1H), 7.54 (dd, J = 8.3, 7.4 Hz, 1H), 7.30 (dd, J = 5.1, 2.9 Hz, 1H), 7.28 (dd, J = 5.1, 1.4 Hz, 1H), 6.80 (dd, J = 7.4, 0.6 Hz, 1H), 5.59 (t, J = 5.4 Hz, 1H), 4.43 (s, 2H), 4.23 (d, J = 5.4 Hz, 2H), 1.53 (s, 9H), 1.47 (s, 9H).

11-(b): Ethyl {6-[(thiophen-3-ylsulfonyl)aminomethyl]pyridin-2-ylamino} acetate

The reaction and post-treatment were performed in accordance with Reference Example 9-(b) with the exception of using 1.63 g (3.37 mmol) of the tert-butyl (tert-butoxycarbonyl{6-[(thiophen-3-ylsulfonyl)aminomethyl]pyridine-2-yl} amino)acet ate obtained in Reference Example 11-(a) in place of tert-butyl (tert-butoxycarbonyl{6-[(thiophen-2-ylsulfonyl)aminomethyl]pyridin-2-ylamino}acetat e, and using 17.5 mL (35.0 mmol) of 2 mol/L hydrogen chloride/ethanol solution. The residue was subjected to silica gel column chromatography (elution solvent: n-hexane:ethyl acetate = 7:3→1:1 (V/V)) and the fractions containing the target product were concentrated under reduced pressure. The resulting crude product was recrystallized with 5 mL of ethyl acetate to obtain 731 mg of the title compound in the form of a white solid (yield: 61%).
mass spectrum (CI, m/z): 356 ($M^+$+1).
$^1$H-NMR spectrum ($CDCl_3$, δ ppm): 7.93 (dd, J = 3.0, 1.4 Hz, 1H), 7.33-7.28 (m, 3H), 6.40 (dd, J = 7.3, 0.6 Hz, 1H), 6.32 (dd, J = 8.3, 0.6 Hz, 1H), 5.76 (t, J = 5.1 Hz, 1H), 4.95 (t, J = 5.4 Hz, 1H), 4.27 (q, J = 7.2 Hz, 2H), 4.13 (d, J = 5.1 Hz, 2H), 4.06 (d, J = 5.4 Hz, 2H), 1.32 (t, J = 7.2 Hz, 3H).

[Reference Example 12]

tert-Butyl [tert-butoxycarbonyl(6-{[3'-(1-propynyl)biphenyl-4-ylmethyl]-aminomethyl}pyridin-2-yl)amino]acetate

12-(a): 1-Bromo-3-(1-propynyl)benzene

1.43 g (7.51 mmol) of cuprous iodide and 1.45 g (1.25 mmol) of tetrakis(triphenylphosphine)palladium were added to 50 mL solution of toluene containing 7.07 g (25.0 mmol) of 1-bromo-3-iodobenzene followed by degassing under reduced pressure and substituting with argon gas. Next, 2.81 g (25.0 mmol) of 1-trimethylsilyl-1-propyne, 11.5 mL (82.5 mmol) of triethylamine and 25.0 mL (25.0 mmol) of 1.0 mol/L tetrafluoroammonium fluoride/tetrahydrofuran solution were added followed by stirring for 17 hours at room temperature in an argon gas atmosphere. After completion of the reaction, water and t-butyl methyl ether were added to the reaction solution and insoluble substances were collected by filtering through Celite (trade name). Following separation, the organic layer was dried with anhydrous magnesium sulfate and then concentrated under reduced pressure. The residue was subjected to silica gel column chromatography (elution solvent: n-hexane) and the fractions containing the target product were concentrated under reduced pressure to obtain 4.22 g of the title compound in the form of a colorless oil (yield: 86%).
mass spectrum (CI, m/z): 195, 197 ($M^+$+1).
$^1$H-NMR spectrum ($CDCl_3$, δ ppm): 7.53 (dd, J = 1.7, 1.7 Hz, 1H), 7.39 (ddd, J = 8.0, 1.7, 1.0 Hz, 1H), 7.31-7.29 (m, 1H), 7.14 (dd, J = 8.0, 8.0 Hz, 1H), 2.04 (s, 3H).

12-(b): 3'-(1-Propynyl)biphenyl-4-ylcarbaldehyde

3.31 g of the title compound were obtained in the form of a pale yellowish white solid by performing the reaction and post-treatment in accordance with Reference Example 5 with the exception of respectively using 2.93 g (15.0 mmol) of 1-bromo-3-(1-propynyl)benzene obtained according to the same method as Reference Example 12-(a) in place of 3-bromophenetole and using 3.37 g (37.5 mmol) of 4-formylphenylboronic acid in place of 4-(hydroxymethyl)phenylboronic acid, and using 11.3 mL (22.6 mmol) of 2 mol/L aqueous sodium carbonate solution and 867 mg (0.750 mmol) of tetrakis(triphenylphosphine)palladium (quantitative).
The NMR spectrum of the compound obtained in the present Reference Example 12-(b) was the same as the NMR spectrum of the compound obtained in Reference Example 4-(a).

12-(c): tert-Butyl [tert-butoxycarbonyl(6-{[3'-(1-propynyl)biphenyl-4-ylmethyl]aminomethyl}pyridin-2-yl)amino]acetate

6.48 g of the title compound was obtained in the form of a pale yellow oil by performing the reaction and post-treatment in accordance with Reference Example 7-(b) with the exception of using 5.57 g (16.5 mmol) of tert-butyl [(6-aminomethylpyridin-2-yl)tert-butoxycarbonylamino] acetate obtained according to the same method as Reference Example 1-(e), 3.30 g (15.0 mmol) of the 3'-(1-propynyl)biphenyl-4-ylcarbaldehyde obtained in Reference Example 12-(b) in place of 3'-ethoxybiphenyl-4-ylcarbaldehyde, and 4.45 g (21.0 mmol) of sodium triacetoxyborohydride (yield: 80%).
mass spectrum (CI, m/z): 542 ($M^+$+1).
$^1$H-NMR spectrum ($CDCl_3$, δ ppm): 7.70 (d, J = 8.2 Hz, 1H), 7.63-7.62 (m, 1H), 7.59 (dd, J = 8.2, 7.4 Hz, 1H), 7.55-7.52 (m, 2H), 7.50-7.47 (m, 1H), 7.43-7.40 (m, 2H), 7.37-7.32 (m, 2H), 6.97 (d, J = 7.4 Hz, 1H), 4.57 (s, 2H), 3.85 (s, 2H), 3.83 (s, 2H), 2.07 (s, 3H), 1.53 (s, 9H), 1.41 (s, 9H).

[Reference Example 13]

3'-(1-Propynyl)biphenyl-4-ylmethanol

A reaction was performed in accordance with Reference Example 5 with the exception of using 3.90 g (20.0

mmol) of 1-bromo-3-(1-propynyl)benzene obtained according to the same method as Reference Example 12-(a) in place of 3-bromophenetole, and using 4.56 g (30.0 mmol) of 4-(hydroxymethyl)phenylboronic acid, 15 mL (30 mmol) of 2 mol/L aqueous sodium carbonate solution and 1.16 g (1.00 mmol) of tetrakis(triphenylphosphine)palladium. After completion of the reaction, water was added to the reaction solution followed by extracting with ethyl acetate. The organic layer was washed with saturated aqueous sodium chloride solution, dried with anhydrous magnesium sulfate and then concentrated under reduced pressure. The residue was subjected to silica gel column chromatography (elution solvent: n-hexane:ethyl acetate = 4:1→1:1 (V/V)) and the fractions containing the target product were concentrated under reduced pressure. The resulting crude product was stirred for 1 hour in 45 mL of a mixed solvent (ethyl acetate:n-hexane = 1:10 (V/V)), and the precipitated solid was collected by filtration and then dried under reduced pressure to obtain 3.85 g of the title compound in the form of a white solid (yield: 87%).

The NMR spectrum of the compound obtained in the present Reference Example 13 was the same as the NMR spectrum of the compound obtained in Reference Example 4-(b).

[Reference Example 14]

Isopropyl {6-[(pyridin-2-ylsulfonyl)aminomethyl]pyridin-2-ylamino}acetate

1.05 g of the title compound were obtained in the form of a white solid by performing the reaction and post-treatment in accordance with Reference Example 9-(b) with the exception of using 1.44 g (3.01 mmol) of tert-butyl (tert-butoxycarbonyl{6-[(pyridin-2-ylsulfonyl)aminomethyl]pyridin-2-ylamino}acetate obtained according to the same method as Reference Example 1-(f) in place of tert-butyl (tert-butoxycarbonyl{6-[(thiophen-2-ylsulfonyl)aminomethyl]pyridin-2-ylamino}acetat e, and using 16.0 mL (32.0 mmol) of 2 mol/L hydrogen chloride/isopropanol solution in place of the 2 mol/L hydrogen chloride/ethanol solution (yield: 96%).
mass spectrum (CI, m/z): 365 ($M^{+}$+1).
$^{1}$H-NMR spectrum ($CDCl_3$, $\delta$ ppm): 8.63 (ddd, J = 4.7, 1.7, 1.0 Hz, 1H), 7.97 (ddd, J = 7.7, 1.0, 1.0 Hz, 1H), 7.84 (ddd, J = 7.7, 7.7, 1.7 Hz, 1H), 7.41 (ddd, J = 7.7, 4.7, 1.0 Hz, 1H), 7.29 (dd, J = 8.2, 7.3 Hz, 1H), 6.44 (d, J = 7.3 Hz, 1H), 6.28 (d, J = 8.2 Hz, 1H), 6.04 (t, J = 5.4 Hz, 1H), 5.10 (sep, J = 6.3 Hz, 1H), 4.93 (t, J = 5.4 Hz, 1H), 4.25 (d, J = 5.4 Hz, 2H), 4.04 (d, J = 5.4 Hz, 2H), 1.28 (d, J = 6.3 Hz, 6H).

[Comparative Example 1]

{6-{(3'-Propylbiphenyl-4-ylmethyl)(pyridin-2-ylsulfonyl)aminomethyl]pyridin-2-ylamino}acetate

This compound is the compound of Example Number 754 of WO2009/113600.

1-(a): 3'-Propylbiphenyl-4-ylcarbaldehyde

1.7 mL of water, 1.63 g (7.68 mmol) of tricalcium phosphate and 675 mg (7.68 mmol) of propylboronic acid were added to 28 mL solution of toluene containing 500 mg (1.91 mmol) of 3'-bromobiphenyl-4-ylcarbaldehyde followed by degassing under reduced pressure and substituting with nitrogen gas. Next, 6.2 mg (0.028 mmol) of palladium acetate and 20.2 mg (0.0563 mmol) of butyl di-1-adamantylphosphine were added followed by stirring for 3 hours at 100°C in a nitrogen gas atmosphere. Post-treatment following completion of the reaction was performed in accordance with Reference Example 5 to obtain 406 mg of the title compound in the form of a pale yellow oil (yield: 86%).
mass spectrum (EI, m/z): 224 ($M^{+}$).
$^{1}$H-NMR spectrum ($CDCl_3$, $\delta$ ppm): 10.06 (s, 1H), 7.99-7.91 (m, 2H), 7.78-7.73 (m, 2H), 7.51-7.34 (m, 3H), 7.28-7.20 (m, 1H), 2.73-2.61 (m, 2H), 1.80-1.62 (m, 2H), 0.98 (t, J = 7.3 Hz, 3H).

1-(b): 3'-Propylbiphenyl-4-ylmethanol

383 mg of the title compound were obtained in the form of a white solid by performing the reaction and post-treatment in accordance with Reference Example 3-(b) with the exception of using 400 mg (1.78 mmol) of the 3'-propylbiphenyl-4-ylcarbaldehyde obtained in Comparative Example 1-(a) in place of 3'-(1-propenyl)biphenyl-4-ylcarbaldehyde, and using 33.7 mg (0.891 mmol) of sodium borohydride (yield: 95%).
mass spectrum (EI, m/z): 226 ($M^{+}$).
$^{1}$H-NMR spectrum ($CDCl_3$, $\delta$ ppm): 7.64-7.55 (m, 2H), 7.48-7.30 (m, 5H), 7.21-7.13 (m, 1H), 4.74 (d, J = 5.6 Hz, 2H), 2.71-2.59 (m, 2H), 1.77-1.62 (m, 3H), 0.97 (t, J = 7.3 Hz, 3H).

1-(c): tert-Butyl (tert-butoxycarbonyl{6-[(3'-propylbiphenyl-4-ylmethyl)

(pyridin-2-ylsulfonyl)aminomethyl]pyridin-2-yl}amino)acetate

255 mg of the title compound were obtained by performing the reaction and post-treatment in accordance with Example 1 with the exception of respectively using 94.6 mg (0.418 mmol) of the 3'-propylbiphenyl-4-ylmethanol obtained in Comparative Example 1-(b) and 200 mg (0.418 mmol) of tert-butyl (tert-butoxycarbonyl {6-[(pyridin-2-ylsulfonyl)aminomethyl]pyridin-2-ylamino}acetate obtained according to the same method as Reference Example 1-(f) in place of 3'-(1-propenyl)biphenyl-4-ylmethanol, and using 198 $\mu$L (0.802 mmol) of tri-n-butylphosphine and 113 mg (0.656 mmol) of N,N,N',N'-tetramethylazodicarboxamide (yield: 89%).
$^1$H-NMR spectrum ($CDCl_3$, $\delta$ ppm): 8.62-8.58 (m, 1H), 7.85-7.73 (m, 2H), 7.65 (d, J = 8.3 Hz, 1H), 7.49-7.23 (m, 9H), 7.20-7.12 (m, 1H), 6.92 (d, J = 7.3 Hz, 1H), 4.73 (s, 2H), 4.52 (s, 2H), 4.46 (s, 2H), 2.69-2.61 (m, 2H), 1.77-1.61 (m, 2H), 1.52 (s, 9H), 1.42 (s, 9H), 0.98 (t, J = 7.3 Hz, 3H).

1-(d): {6-[(3'-Propylbiphenyl-4-ylmeth)(pyridin-2-ylsulfonyl)aminomethyl]-pyridin-2-ylamino}acetate

0.74 mL (9.7 mmol) of trifluoroacetic acid was added at room temperature to 1.7 mL solution of methylene chloride containing 247 mg (0.360 mmol) of the tert-butyl (tert-butoxycarbonyl {6-[(3'-propylbiphenyl-4-ylmethyl)(pyridin-2-ylsulfonyl)aminomethyl]pyridin-2-yl}amino)acetate obtained in Comparative Example 1-(c) followed by stirring for 20 hours at room temperature. After completion of the reaction, the reaction solution was concentrated under reduced pressure followed by addition of water and adjusting the pH to 4.5 with 1 mol/L aqueous sodium hydroxide solution and 1 mol/L hydrochloric acid. The precipitated solid was collected by filtration, washed and concentrated under reduced pressure to obtain 161 mg of the title compound in the form of a white solid (yield: 84%).
mass spectrum (FAB, m/z): 531 ($M^+$+1).
$^1$H-NMR spectrum (DMSO-$d_6$, $\delta$ ppm): 12.42 (brs, 0.8H), 8.66-8.63 (m, 1H), 7.95 (ddd, J = 7.7, 7.6, 1.5 Hz, 1H), 7.83-7.79 (m, 1H), 7.58 (ddd, J = 7.6, 4.7, 0.8 Hz, 1H), 7.57-7.53 (m, 2H), 7.46-7.41 (m, 2H), 7.38-7.34 (m, 1H), 7.33-7.30 (m, 2H), 7.25-7.16 (m, 2H), 6.78 (brs, 0.8H), 6.36 (d, J = 8.1 Hz, 1H), 6.30 (d, J = 7.0 Hz, 1H), 4.73 (s, 2H), 4.26 (s, 2H), 3.84 (s, 2H), 2.65-2.60 (m, 2H), 1.68-1.60 (m, 2H), 0.92 (t, J = 7.3 Hz, 3H).

[Test Example 1]

Measurement of EP2 Receptor Binding Action

Measurement of EP2 receptor binding action was performed in accordance with the method of Abramovitz et al. (Biochimica et Biophysica Acta, 1483,285 (2000)). A test compound dissolved in dimethyl sulfoxide (final concentration: 1.0 (VN)%) and [$^3$H]prostaglandin $E_2$ (NET-428, manufactured by PerkinElmer) (final concentration: 10 nM) were added to a buffer solution (10 mM MES-KOH (pH 6.0), 10 mM $MgCl_2$, 1 mM EDTA) in which 10 $\mu$g of a membrane fraction of HEK293 cells expressing human EP2 receptor (ES-562-M, manufactured by Euroscreen) was suspended, and then incubated at 30°C for 60 minutes. The membrane fraction was collected on glass fiber filter paper (GF/B, manufactured by Whatman) using a cell harvester (M30R, manufactured by Brandel), and after washing with a buffer solution (10 mM MES-KOH (pH 6.0), 10 mM $MgCl_2$), radioactivity was measured with a liquid scintillation analyzer (2000CA, manufactured by Packard). The concentration of test compound required to replace 50% of the [$^3$H]prostaglandin $E_2$ bound to the receptor ($IC_{50}$ value) was calculated using EXSAS (Ver. 7.1.6, manufactured by Arm Systex), and the inhibition constant (Ki value) was determined using the formula indicated below.

$$Ki = IC_{50} / (1 + ([^3H]\text{prostaglandin } E_2 \text{ concentration} / Kd))$$

Furthermore, the dissociation constant (Kd value) was calculated by Scatchard analysis.
The test results are shown in Table 1.

[Table 1]

| Test compound no. | Ki value (nM) of EP2 receptor binding action |
|---|---|
| Example 2 | 0.53 |
| Example 4 | 0.80 |
| Example 6 | 0.75 |

(continued)

| Test compound no. | Ki value (nM) of EP2 receptor binding action |
|---|---|
| Example 7 | 0.61 |
| Example 9 | 0.80 |
| Example 10 | 0.97 |
| Example 11 | 0.79 |
| Example 12 | 0.94 |
| Example 14 | 0.90 |
| Example 16 | 0.95 |
| Example 18 | 0.99 |
| Comparative Example 1 | 1.10 |

In the present test, compounds of the present invention demonstrated superior EP2 receptor binding action.

[Test Example 2]

Test of Inhibition of LPS-Induced TNFα Production Using Human Peripheral Blood Mononuclear Cells

Peripheral blood collected in the presence of heparin from healthy subjects was diluted two-fold with PBS containing 2 (V/V)% FBS. Hemocyte separation solution (Ficoll Paque™, manufactured by GE Healthcare Science) was added to SepMate™-50 (manufactured by STEMCELL Technologies Inc.) followed by layering the diluted blood thereon. A peripheral blood mononuclear cell (hereinafter abbreviated as PBMC) layer was recovered by centrifuging for 10 minutes under conditions of 20°C and 1200xg. The resulting PBMCs were further centrifuged and washed twice followed by suspending in RPMI1640 medium containing 1 (V/V)% FBS for use in the following test.

A test of inhibition of LPS-induced TNFα production was performed by partially modifying the method of Mary et al. (Journal of Pharmacology and Experimental Therapeutics, 284, 420 (1998)). 185 μL of PBMC suspension prepared to a final concentration of $5 \times 10^5$ cells/mL was added to a 96-well plate, subsequently 10 μL of RPMI1640 medium, containing 1 (V/V)% DMSO in which a test compound was dissolved, was added to each well (DMSO final concentration: 0.05 (V/V)%). RPMI1640 medium containing 1 (V/V)% DMSO was similarly added to a well to which test compounds had not been added. After incubating for 1 hour in a carbon dioxide gas incubator, 5 μL of RPMI1640 medium containing LPS (L2880-500MG, manufactured by Sigma) was added to each well (LPS final concentration: 100 ng/mL). 5 μL of RPMI1640 medium was added to a non-LPS stimulation well. After culturing for about 18 hours in a carbon dioxide gas incubator, the culture supernatant was recovered. The recovered culture supernatant was stored at -20°C until measurement of TNFα content.

A sandwich ELISA kit (Quantikine DTA00c, manufactured by R&D Systems) was used to measure TNFα content. The TNFα content of each sample was calculated from a standard curve of E. coli-derived human recombinant TNFα provided with the kit. TNFα production inhibition rate at each test compound concentration was calculated based on a value of 100% for the amount of TNFα produced by LPS in the case of only adding DMSO. The concentration of test compound that inhibited TNFα production by 50% was calculated as the $IC_{50}$ value (nM) from the relationship between concentration of the added test compound and TNFα production inhibition rate of the test compound.

The test results are shown in Table 2.

[Table 2]

| Test compound no. | $IC_{50}$ value (nM) of TNFα production inhibitory action of human PBMC |
|---|---|
| Example 4 | 3.3 |
| Example 6 | 9.4 |
| Example 7 | 20 |
| Example 10 | 18 |
| Example 11 | 23 |
| Example 12 | 16 |

(continued)

| Test compound no. | $IC_{50}$ value (nM) of TNFα production inhibitory action of human PBMC |
|---|---|
| Example 14 | 13 |
| Example 16 | 15 |
| Example 18 | 3.2 |
| Comparative Example 1 | >300 |

In the present test, compounds of the present invention demonstrated superior inhibitory action on production of TNFα.

[Test Example 3]

Inhibitory Effect on Neutrophil Infiltration of Rat Lung

A test of inhibition of neutrophil infiltration of the rat lung was performed by partially modifying the method of Spond et al. (Pulmonary Pharmacology and Therapeutics, 14, 157 (2001)). 25 μL (approx. 4 μg/Kg) of physiological saline solution containing LPS (L2880-500MG, manufactured by Sigma) (concentration: 0.04 mg/mL) was administered intratracheally under isoflurane inhalation anesthesia into SD rats fasted for about 16 hours (males, 7-8 weeks old, body weights: 240 g to 270 g (average: approx. 250 g), supplied by Charles River Laboratories, Japan). A Microsprayer™ (IA-1C-M, manufactured by PennCentury) was used for intratracheal administration.

Dosing solutions of the test compounds were prepared by dissolving the test compounds in a 0.1 mol/L or 1 mol/L aqueous sodium hydroxide solution followed by adding medium and neutralizing (final test compound concentration: 1 mg/mL). PBS or phosphate buffer solution (20 mM, pH = 7.4) was used for the medium. 25 μL (approx. 0.1 mg/Kg) of the test compound solution prepared in this manner was administered intratracheally in the same manner as LPS administration 1 hour prior to administration of LPS. Medium was administered to a control group. Furthermore, 6 animals were used in the test compound dosing groups and control group, respectively.

Bronchoalveolar lavage was performed in the manner described below 4 hours after administration of LPS followed by collection of white blood cells present in the lungs. The SD rats were anesthetized by intraperitoneal administration of somnopentyl (1 mL/Kg) and then exsanguinated by severing the inferior vena cava. After exposing the trachea and inserting a mouse feeding needle (manufactured by Fuchigami Kikai) connected to a disposable syringe (5 mL, manufactured by Terumo), the trachea was immobilized by ligation. 4 mL of physiological saline solution containing BSA (final concentration: 1%) and heparin (final concentration: 1 U/mL) were injected and then immediately collected to obtain bronchoalveolar lavage fluid (BALF). This procedure was then repeated twice, and after centrifuging the resulting BALF (420xg, 10 minutes, 4°C), supernatant was removed until the amount of liquid became 1.5 mL followed by suspending the precipitated cells to obtain a BALF cell suspension. Measurement of the numbers of white blood cells and neutrophils in the BALF cell suspension was performed according to either (Method 1) or (Method 2) described below.

(Method 1)

The number of white blood cells present in the BALF suspension was measured using a multiparameter automated hematology analyzer (KX-21, manufactured by Sysmex). Next, the BALF suspension was diluted so that the number of white blood cells was $10^6$ cells/mL, and 100 μL of this cell suspension was then applied to a slide glass to prepare a thin layer smear. Next, after staining the cells using the Diff-Quik staining kit (Cat. No. 16920, manufactured by Sysmex), the number of neutrophils present among 300 white blood cells was measured under a light microscope (BH-2, manufactured by Olympus) followed by calculating the ratio of neutrophils to white blood cells (NR = the number of neutrophils among 300 white blood cells/300). The inhibition rate of neutrophil infiltration following administration of a test compound was calculated according to the equation indicated below.

$$\text{Inhibition rate (\%)} = 100 - [(\text{WBCc} \times \text{NRc})/(\text{WBCv} \times \text{NRv})] \times 100$$

WBCv: Number of white blood cells in BALF cell suspension of control group
WBCc: Number of white blood cells in BALF cell suspension of test compound dosing group
NRv: Ratio of neutrophils to white blood cells of control group

NRc: Ratio of neutrophils to white blood cells of test compound dosing group

(Method 2)

The number of neutrophils present in the BALF cell suspension was measured using a multiparameter automated hematology analyzer (XT-2000iV, manufactured by Sysmex). The inhibition rate of neutrophil infiltration following administration of a test compound was calculated according to the equation indicated below.

$$\text{Inhibition rate (\%)} = 100 - [(\text{NEUTc})/(\text{NEUTv})] \times 100$$

NEUTv: Number of neutrophils in BALF cell suspension of control group
NEUTc: Number of neutrophils in BALF cell suspension of test compound dosing group

In the present test, the test compounds of the present invention demonstrated superior inhibitory action on infiltration of the lungs by neutrophils. For example, the compounds of Examples 4, 14 and 16 inhibited plumonary neutrophil infiltration by 71%, 65% and 66%, respectively.
Representative preparation examples used in the present invention are indicated below.

(Preparation Example 1) (Hard Capsule)

50 mg of the compound of Example 2 in powdered form, 128.7 mg of lactose, 70 mg of cellulose and 1.3 mg of magnesium stearate are mixed and passed through a 60-mesh sieve followed by placing 250 mg of this powder in a No. 3 gelatin capsule to obtain a capsule preparation.

(Preparation Example 2) (Tablet)

50 mg of the compound of Example 2, 124 mg of lactose, 25 mg of cellulose and 1 mg of stearic acid are mixed and formed into a tablet with a tablet-making machine to obtain a tablet weighing 200 mg per tablet. This tablet can be provided with a sugar coating as necessary.

INDUSTRIAL APPLICABILITY

The substituted biaryl compound represented by general formula (I), or the pharmacologically acceptable salt thereof, of the present invention is effective for chronic obstructive pulmonary disease due to having an EP2 antagonistic effect and an excellent anti-inflammatory effect. Thus, a pharmaceutical composition containing the substituted biaryl compound represented by general formula (I), or the pharmacologically acceptable salt thereof, of the present invention as an active ingredient thereof is useful as pharmaceuticals, and especially as a therapeutic and/or prophylactic drug (and preferably as a therapeutic drug) for chronic obstructive pulmonary disease.

**Claims**

1. A pharmaceutical composition for treatment and/or prevention of chronic obstructive pulmonary disease, comprising a substituted biaryl compound represented by general formula (I):

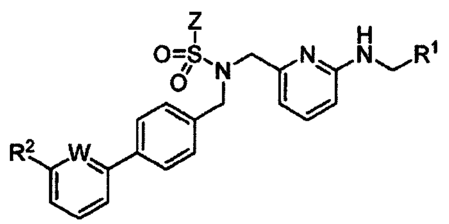

(I)

wherein,

R$^1$ represents a protected or unprotected carboxy group,

W represents a nitrogen atom or -CH= group,
$R^2$ represents an ethoxy group, 1-propenyl group or 1-propynyl group, and
Z represents a phenyl group, 3-fluorophenyl group, pyridin-2-yl group, pyridin-3-yl group, thiophen-2-yl group or thiophen-3-yl group

or a pharmacological acceptable salt thereof.

2. The pharmaceutical composition according to claim 1, wherein, $R^1$ represents a carboxy group or $C_1$-$C_6$ alkoxycarbonyl group.

3. The pharmaceutical composition according to claim 1, wherein $R^1$ represents a carboxy group, ethoxycarbonyl group, isopropoxycarbonyl group or hexyloxycarbonyl group.

4. The pharmaceutical composition according to claim 1, wherein
$R^1$ represents a carboxy group, ethoxycarbonyl group, isopropoxycarbonyl group or hexyloxycarbonyl group,
W represents a nitrogen atom or -CH= group,
$R^2$ represents a 1-propenyl group or 1-propynyl group, and
Z represents a phenyl group, 3-fluorophenyl group, pyridin-2-yl group, pyridin-3-yl group, thiophen-2-yl group or thiophen-3-yl group.

5. The pharmaceutical composition according to claim 1, wherein the substituted biaryl compound represented by general formula (I) is:

ethyl (6-{[3'-(1-propenyl)biphenyl-4-ylmethyl](pyridin-2-ylsulfonyl)aminomethyl}pyridin-2-ylamino)acetate,
(6-{[3'-(1-propenyl)biphenyl-4-ylmethyl](pyridin-2-ylsulfonyl)aminomethyl}-pyridin-2-ylamino)acetic acid,
ethyl (6-{[3'-(1-propynyl)biphenyl-4-ylmethyl](pyridin-2-ylsulfonyl)aminomethyl}pyridin-2-ylamino)acetate,
(6-{[3'-(1-propynyl)biphenyl-4-ylmethyl](pyridin-2-ylsulfonyl)aminomethyl}-pyridin-2-ylamino)acetic acid,
ethyl (6-{[3'-(1-propynyl)biphenyl-4-ylmethyl](pyridin-3-ylsulfonyl)aminomethyl}pyridiN-2-ylamino)acetate,
(6-{[3'-(1-propynyl)biphenyl-4-ylmethyl](pyridin-3-ylsulfonyl)aminomethyl}-pyridin-2-ylamino)acetic acid,
{6-[(3'-ethoxybiphenyl-4-ylmethyl)(pyridin-2-ylsulfonyl)aminomethyl]-pyridin-2-ylamino} acetic acid,
hexyl {6-[(3'-ethoxybiphenyl-4-ylmethyl)(pyridin-2-ylsulfonyl)aminomethyl]-pyridin-2-ylamino acetate,
{6-[(3'-ethoxybiphenyl-4-ylmethyl)(pyridin-3-ylsulfonyl)aminomethyl]-pyridin-2-ylamino} acetic acid,
{6-[(benzenesulfonyl)(3'-ethoxybiphenyl-4-ylmethyl)aminomethyl]pyridin-2-ylamino} acetic acid,
{6-[(3'-ethoxybiphenyl-4-ylmethyl)(thiophen-2-ylsulfonyl)aminomethyl]-pyridin-2-ylamino} acetic acid,
(6-{[4-(6-ethoxypyridin-2-yl)benzyl] (pyridin-2-ylsulfonyl)aminomethyl}-pyridin-2-ylamino)acetic acid,
ethyl (6-{[3'-(1-propynyl)biphenyl-4-ylmethyl] (thiophen-2-ylsulfonyl)-aminomethyl}pyridin-2-ylamino)acetate,
(6-{[3'-(1-propynyl)biphenyl-4-ylmethyl](thiophen-2-ylsulfonyl)-aminomethyl}pyridin-2-ylamino)acetic acid,
ethyl (6-{(benzenesulfonyl)[3'-(1-propynyl)biphenyl-4-ylmethyl]-aminomethyl}pyridin-2-ylamino)acetate,
(6-{(benzenesulfonyl)[3'-(1-propynyl)biphenyl-4-ylmethyl]aminomethyl}-pyridin-2-ylamino)acetic acid,
ethyl (6-{[3'-(1-propynyl)biphenyl-4-ylmethyl](thiophen-3-ylsulfonyl)-aminomethyl}pyridin-2-ylamino)acetate,
(6-{[3'-(1-propynyl)biphenyl-4-ylmethyl](thiophen-3-ylsulfonyl)-aminomethyl}pyridin-2-ylamino)acetic acid,
(6-{(3-fluorobenzenesulfonyl)[3'-(1-propynyl)biphenyl-4-ylmethyl]-aminomethyl}pyridin-2-ylamino)acetic acid,
or
isopropyl (6-{[3'-(1-propynyl)biphenyl-4-ylmethyl](pyridin-2-ylsulfonyl)aminomethyl}pyridin-2-ylamino)acetate.

## INTERNATIONAL SEARCH REPORT

International application No.
PCT/JP2014/072987

A. CLASSIFICATION OF SUBJECT MATTER
*A61K31/44*(2006.01)i, *A61K31/4436*(2006.01)i, *A61K31/444*(2006.01)i, *A61P11/00*(2006.01)i

According to International Patent Classification (IPC) or to both national classification and IPC

B. FIELDS SEARCHED

Minimum documentation searched (classification system followed by classification symbols)
A61K31/44, A61K31/4436, A61K31/444, A61P11/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2009/113600 A1 (Ube Industries, Ltd.), 17 September 2009 (17.09.2009), entire text; particularly, claims; page 81<br>& JP 5136640 B2 & JP 2013-63994 A<br>& US 2011/0054172 A1 & US 2014/0113907 A1<br>& EP 2264009 A1 & AU 2009224329 A<br>& CA 2718393 A & CN 101970410 A<br>& KR 10-2010-0123903 A & CN 103965099 A | 1-3,5<br>4 |
| A | WO 2011/030864 A1 (Ube Industries, Ltd.), 17 March 2011 (17.03.2011), entire text<br>& US 2012/0184747 A1 & EP 2476668 A1<br>& CA 2773997 A & CN 102498101 A<br>& AU 2010293406 A & KR 10-2012-0068902 A | 1-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>27 November, 2014 (27.11.14) | Date of mailing of the international search report<br>09 December, 2014 (09.12.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2014/072987

C (Continuation). DOCUMENTS CONSIDERED TO BE RELEVANT

| Category* | Citation of document, with indication, where appropriate, of the relevant passages | Relevant to claim No. |
|---|---|---|
| A | WO 2011/030871 A1 (Ube Industries, Ltd.), 17 March 2011 (17.03.2011), entire text (Family: none) | 1-5 |
| A | Lars Treffkorn et al., PGE2 exerts its effect on the LPS-induced release of TNF-α, ET-1, IL-1α, IL-6 and IL-10 via the EP2 and EP4 receptor in rat liver macrophages, Prostaglandins & Other Lipid Mediators, 2004, Volume 74, Issues 1-4, Pages 113-123 | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006043655 A
- WO 2009113600 A
- WO 2011030868 A
- WO 2011030871 A
- WO 2011030872 A
- WO 2011030873 A
- WO 2011078303 A
- US 2003199440 A


### Non-patent literature cited in the description


- *British Journal of Pathology,* 1997, vol. 122, 149
- *Journal of Organic Chemistry,* 2003, vol. 68, 247
- **ABRAMOVITZ et al.** *Biochimica et Biophysica Acta,* 2000, vol. 1483, 285
- **SPOND et al.** *Pulmonary Pharmacology and Therapeutics,* 2001, vol. 14, 157